# FASCICULE DE BREVET EUROPEEN

(11) **EP 0 406 111 B1**
(45) Date de publication et mention de la délivrance du brevet: **09.10.1996**
(21) Numéro de dépôt: 90401850.4
(22) Date de dépôt: 27.06.1990
(51) Int. Cl.: C07D 409/04, C07D 333/58, C07D 307/81, C07D 409/08, C07D 409/14, C07D 295/02, A61K 31/445, A61K 31/38

(54) **Arylamines et hétéroarylamines substituées, leur procédé de préparation et composition pharmaceutique les contenant**
Substituierte Arylamine und Heteroarylamine, Verfahren zu deren Herstellung und sie enthaltende pharmazeutische Zusammensetzung
Substituted arylamines and heteroarylamines, process for their preparation and pharmaceutical composition containing them

(30) Priorité: 29.06.1989 FR 8908704
(43) Date de publication de la demande: 02.01.1991
(73) Titulaire: SOCIETE DE CONSEILS DE RECHERCHES ET D'APPLICATIONS SCIENTIFIQUES (S.C.R.A.S.), F-75016 Paris (FR)
(72) Inventeur: Kamenka, Jean-Marc, F-34090 Montpellier (FR); Privat, Alain, F-34090 St Clement La Riviere (FR); Chicheportiche, Robert Rubin, F-34070 Montpellier (FR); Costentin, Jean, F-76000 Rouen (FR)
(74) Mandataire: Des Termes, Monique

(56) Documents cités:
- EP-A- 0 317 953
- CHEMICAL ABSTRACTS, vol. 110, no. 17, 24 avril 1989, page 78, résumé no. 147750t, Columbus, Ohio, US; J.M. SANI et al.: "Interactions of phencyclidine and two derivatives with serotonergic receptors"
- CHEMICAL ABSTRACTS, vol. 109, no. 3, 18 juillet 1988, page 44, résumé no. 16882u, Columbus, Ohio, US; J. VIGNON et al.: "[3]N-[1-(2- Benzo(b)thiophenyl)cyclohex y]piperidine [3H]BTCP): a new phencyclidine analog selective for the dopamine uptake complex"
- CHEMICAL ABSTRACTS, vol. 110, no. 23, 5 juin 1989, page 53, résumé no. 205446m, Columbus, Ohio, US; M. SLIMANI et al.: "Neurochemical and behavioral evidence for a central indirect dopaminergic agonist activity of GK 13, a phencyclidine derivative"

## Description

La présente invention a pour objet de nouveaux composés ayant une structure proche de celle du (benzo(b)thiophényl-2)-1 (pipéridino -1) cyclohexane (BTCP) de formule :

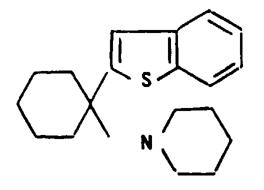

Le BTCP est une molécule dérivée de la phencyclidine, qui présente des propriétés différentes de cette dernière grâce à la présence du noyau benzothiophényle.

La phencyclidine (PCP), c'est-à-dire la (phényl-1 cyclohexyl)-N pipéridine de formule : 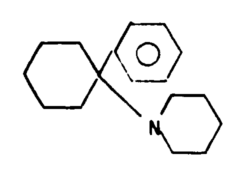 a été synthétisée et étudiée pour ses propriétés pharmacologiques vers 1958.

A la suite de ces études, elle a été introduite comme analgésique et anesthésique sous le nom de Sernyl® et abandonnée ensuite en raison de ses effets secondaires de type psychodysleptique.

La phencyclidine ainsi que la plupart de ses analogues ou dérivés présentent dans leur spectre pharmacologique comportemental une composante dopaminergique improprement dénommée amphétaminique par certains auteurs. En effet, il a pu être montré que cette composante correspondait à une inhibition de la recapture de la dopamine beaucoup plus qu'à une stimulation de libération propre aux amphétamines. La phencyclidine a donc une action dopaminergique indirecte.

La BTCP qui se différencie de la PCP par la présence d'un noyau benzothiophényle à la place du noyau phényle présente des propriétés différentes comme il a été décrit par J. Vignon et al dans European Journal of Pharmacology, 148, (1988), p. 427-436.

En effet, la BTCP possède à un degré beaucoup plus élevé la capacité d'inhiber la recapture de la dopamine "in vitro" tandis que son affinité pour le récepteur de la PCP est très faible. Cette propriété rend la BTCP intéressante pour d'autres applications, par exemple dans le domaine de l'antidépression et de la vigilance.

De plus, il a été démontré par M. Slimani et al dans Domino, E.F. et Kamenka, J.M. (eds), Sigma and Phencyclidin like Compounds as Molecular Probes in Biology, NPP Books, Ann Arbor, 1988, p. 511-520, que la BTCP avait également une action inhibitrice du même type sur la recapture de la noradrénaline.

Aussi, des recherches ont été effectuées pour trouver des molécules du même type possédant l'action dopaminergique la plus importante possible mais aussi et surtout une affinité pour le récepteur de la PCP aussi réduite que possible.

La présente invention a précisément pour objet de nouvelles amines substituées, qui présentent ces propriétés intéressantes.

Selon l'invention, les nouvelles amines substituées répondent à la formule : 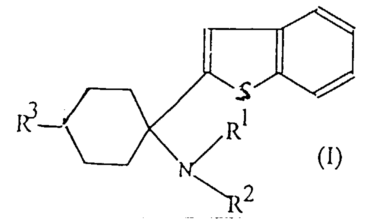 dans laquelle R¹ et R² qui peuvent être identiques ou différents, représentent un radical alkyle ou un radical alkyle substitué par au moins un substituant choisi parmi les atomes d'halogène, les radicaux alcoxy et le radical hydroxyle, ou R¹ et R² forment avec l'atome d'azote auquel ils sont liés, un cycle pipéridine comprenant éventuellement un ou plusieurs substituants choisis parmi le groupe méthyle, le groupe méthyle substitué par un atome d'halogène et le groupe bivalent 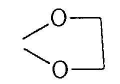 ou = 0 ou dans laquelle R¹ et R² forment avec l'atome d'azote auquel ils sont liés un cycles pipérazine de formule : 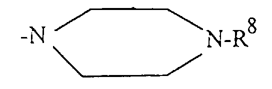 dans laquelle R⁸ représente H, un radical alkyle ou un radical alkyle substitué par au moins un substituant choisi parmi les atomes d'halogène et les groupes hydroxy, alcoxy arylalcoxy et oxycarbonylalkyle, et
- R³ est un atome d'hydrogène ou le groupe méthyle, à condition que R³ ne représente pas H lorsque R¹ et R² forment avec l'atome d'azote auquel ils sont liés un cycle pipéridine non substitué.

Les amines décrites ci-dessus se différencient de la BTCP soit par la présence de substituants sur le noyau cyclohexyle et/ou pipéridine, soit par le remplacement du noyau pipéridine par des radicaux alkyle.

Grâce à ces modifications, on améliore de façon importante le pouvoir inhibiteur de recapture de la dopamine et/ou de la noradrénaline tout en ayant une affinité faible pour le site de la PCP.

Lorsque les substituants utilisés dans l'invention sont des radicaux alkyle, il peut s'agir de radicaux alkyle linéaires ou ramifiés ayant de préférence de 1 à 4 atomes de carbone.

Pour R¹ et R², de bons résultats sont obtenus avec 2 à 3 atomes de carbone.

Lorsque les substituants utilisés dans l'invention sont des radicaux aralkyle, la partie alkyle de ces radicaux a de préférence de 1 à 3 atomes de carbone. A titre d'exemple de tels radicaux, on peut citer les radicaux benzyle et diphénylméthyle.

Dans certains cas, les radicaux alkyle utilisés pour R¹, R² ou comme substituants d'un cycle pipéridine ou pipérazine, peuvent être substitués par au moins un substituant choisi parmi les atomes d'halogène et les groupes alcoxy, hydroxy, aralkoxy et oxycarbonylalkyle. Les radicaux oxycarbonylalkyle peuvent répondre à la formule R-COO- dans laquelle R est un alkyle.

Les halogènes utilisables sont le fluor, le chlore, le brome et l'iode.

Les radicaux alcoxy utilisés peuvent être linéaires ou ramifiés et comporter de 1 à 3 atomes de carbone.

A titre d'exemples de tels radicaux, on peut citer les radicaux méthoxy, éthoxy, propyloxy.

Dans les radicaux oxycarbonylalkyle, le radical alkyle peut être également linéaire ou ramifié et comporte de préférence de 1 à 3 atomes de carbone.

A titre d'exemple de tels radicaux, on peut citer les radicaux suivants : CH₃-COO, C₂H₅COO, C₃H₇COO.

A titre d'exemples de radicaux alkyle substitués utilisés comme substituants d'un cycle pipéridine ou pipérazine, on peut citer -CH₂CH₂OH, -CH₂CH₂O CH₂CH₂CH₃ et -CH₂CH₂OCH(C₆H₅)₂. Lorsqu'il s'agit d'un cycle pipérazine avec R⁸ représentant un radical alkyle substitué, 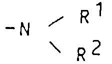 peut répondre par exemple aux formules suivantes : 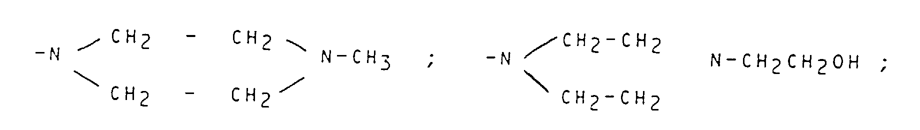 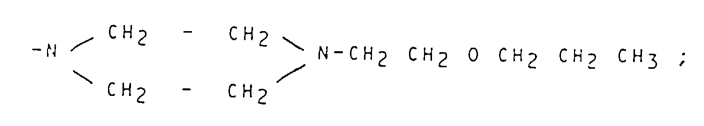 ou

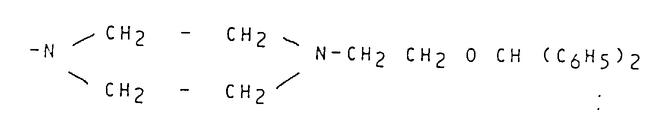

De même, lorsque 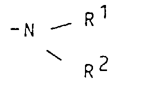 représente un cycle pipéridine comportant un substituant OH, de préférence celui-ci n'est pas en position α par rapport à N.

Dans l'invention, on obtient de bons résultats lorsque R¹ et R² représentent un cycle pipéridine et que le cycle pipéridine est substitué en position 3 et/ou le noyau cyclohexyle est substitué en position 4 par un radical alkyle, de préférence un radical méthyle.

A titre d'exemple de telles amines substituées, on peut citer le (benzo(b) thiophényl-2)-1 c-méthyl-4-r-(pipéridino-1)-1 cyclohexane, le (benzo(b)thiophényl-2)-1 (méthyl-3 pipéridino)-1)-1 cyclohexane, le (benzo(b)thiophényl-2)-1 (diméthyl-3,5 pipéridino)-1)-1 cyclohexane.

Selon l'invention, on obtient également de très bons résultats lorsque R¹ et R² sont des radicaux alkyle, par exemple des radicaux propyle ou éthyle. Dans ce cas, il n'est pas nécessaire que le noyau cyclohexyle soit substitué par un groupement méthyle.

A titre d'exemple de telles amines substituées, on peut citer le (benzo(b)thiophényl-2)-1 (dipropylamino-1)-1 cyclohexane.

Les amines substituées de l'invention peuvent être préparées par différents procédés.

Dans le cas des amines substituées de formule (I) 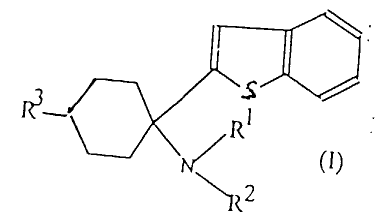 dans laquelle R¹ et R² qui peuvent être identiques ou différents, représentent un radical alkyle ou un radical alkyle substitué par au moins un substituant choisi parmi les atomes d'halogène, les radicaux alcoxy et le radical hydroxy, ou R¹ et R² forment avec l'atome d'azote auquel ils sont liés, un cycle pipéridine comprenant éventuellement un ou plusieurs substituants choisis parmi le groupe méthyle, le groupe méthyle substitué par au moins un atome d'halogène et le groupe bivalent 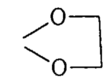 ou = 0 ou dans laquelle R¹ et R² forment avec l'atome d'azote auquel ils sont liés un cycle pipérazine de formule : 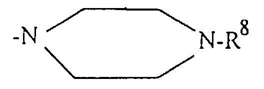 dans laquelle R⁸ représente H, un radical alkyle ou un radical alkyle substitué par au moins un substituant choisi parmi les atomes d'halogène et les groupes hydroxy, alcoxy, arylalcoxy et oxycarbonylalkyle, et
- R³ est un atome d'hydrogène ou le groupe méthyle, à condition que R³ ne représente pas H lorsque n est égal à O, et lorsque R¹ et R² forment avec l'atome d'azote auquel ils sont liés un cycle pipéridine non substitué, on peut utiliser un procédé consistant à :
   a) préparer un α-aminonitrile de formule : 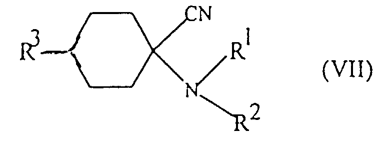 dans laquelle R¹, R² et R³ ont la signification donnée ci-dessus, et
   b) faire réagir l'α-aminonitrile ainsi préparé avec un halogénure de formule:MgXR⁵ dans laquelle X représente un atome d'halogène sauf le fluor, et R⁵ représente le groupe benzo thiophényle-2.

Lorsque 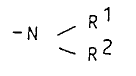 représente 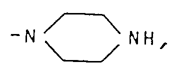 on protège avant l'étape a) le groupe NH du cycle pipérazine de l'α-aminonitrile de formule (VII) par un radical approprié tel que le radical acétyle, et on réalise la déprotection de ce groupe NH après l'étape b).

Selon ce procédé, on peut préparer les alpha-aminonitriles de formule (VII) à partir des cyclohexanones de formule (VIII) : 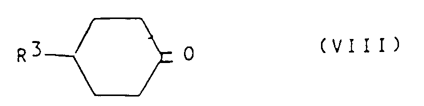 dans laquelle R³ a la signification donnée ci-dessus avec une amine ou une pipéridine de formule (IX) : 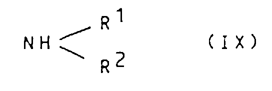 dans laquelle R¹ et R² ont la signification donnée ci-dessus.

Pour cette préparation, on peut faire réagir de l'acétone cyanhydrine (donneur d'ions cyanure) avec l'amine ou la pipéridine de formule (IX), et la cyclohexanone de formule (VIII), en présence de sulfate de magnésium qui agit comme déshydratant, dans un solvant tel que le diméthylacétamide (DMA). Après traitement et purification, on isole l'alpha-aminonitrile correspondant avec une pureté d'au moins 95% et on peut l'utiliser tel quel pour la deuxième étape du procédé.

Dans la deuxième étape, on fait réagir cet alpha-aminonitrile de formule (VII) avec l'halogénure de formule MgXR⁵ en utilisant la synthèse dite de Bruylants.

Cette synthèse consiste à faire réagir l'alpha-aminonitrile avec un réactif de Grignard obtenu à partir de l'halogénure XR⁵ dans l'éther ou le tétrahydrofuranne (THF) anhydre. Après traitement convenable et purification, on isole l'amine substituée recherchée. On fait ensuite barboter du gaz chlorhydrique anhydre dans une solution éthérée de l'amine substituée pure, et on précipite ainsi le chlorhydrate hydrosoluble correspondant.

On peut préparer d'autres sels d'addition aux acides en remplaçant le gaz chlorhydrique par l'acide voulu, par exemple l'acide sulfurique ou tartrique.

Cette voie de synthèse est stéréospécifique ; elle ne permet donc d'accéder qu'à un seul des isomères possibles.

Les amines substituées obtenues par ce procédé dans lesquelles R¹ et R² forment avec l'atome d'azote auquel ils sont liés, un cycle pipéridine comprenant un ou plusieurs substituants constitués par des radicaux méthyle substitués par un groupe hydroxyle, peuvent être utilisés pour la préparation d'amines substituées conformes à l'invention dans lesquelles R¹ et R² forment avec l'atome d'azote auquel ils sont liés, un cycle pipéridine comprenant un ou plusieurs substituants constitués par des radicaux méthyle substitués par un atome d'halogène.

Dans ce cas, on fait réagir les amines substituées hydroxyméthylées avec un agent halogénant approprié pour remplacer le groupe hydroxyle par un atome d'halogène.

Lorsque l'atome d'halogène est le brome, on peut utiliser comme agent halogénant du bromure de thionyle ; Lorsque l'atome d'halogène est du chlore, on peut utiliser le chlorure de thionyle ; lorsque l'atome d'halogène est l'iode, on peut utiliser l'iodure de triméthylsilane.

On peut aussi préparer les amines substituées de formule (I) de l'invention dans lesquelles R¹ et R² forment avec l'atome d'azote auquel ils sont liés un cycle pipéridine non substitué ou substitué par au moins un substituant choisi parmi le groupes méthyle et le groupe méthyle substitué par un atome d'halogène par un procédé en 4 étapes qui présente en particulier l'avantage de ne pas être stéréospécifique et de permettre l'accès aux deux isomères possibles.

Ce procédé consiste :
a) à préparer un alcool de formule : 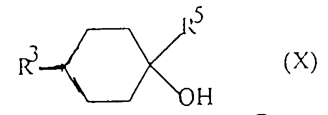 dans laquelle R³ a la signification donnée ci-dessus et R⁵ représente le groupe benzothiophényle-2,
b) à transformer l'alcool préparé dans l'étape a) en un dérivé azide correspondant de formule : 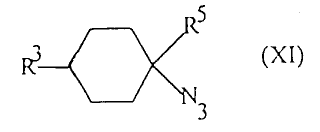
c) à réduire le dérivé azide en un mélange d'amines primaires, et
d) à faire réagir le mélange d'amine primaires avec un dihalogéno-1,5 pentane comportan éventuellement un ou plusieurs substituants choisi parmi le groupe méthyle et le groupe méthyle substitu par un atome d'halogène.

Cette synthèse se déroule ainsi en 4 étapes qui sont les suivantes :
a) Préparation de l'alcool de formule (X).
   Ceci peut être effectué en faisant réagir la cyclohexanone de formule (VIII) 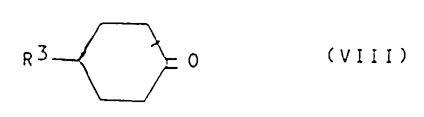 avec un réactif de Grignard obtenu à partir des halogénures de formule XR⁵ dans de l'éther anhydre. On obtient ainsi les alcools correspondants que l'on soumet à une rapide purification.
b) Préparation des dérivés azide correspondants de formule (XI).
   Ceci peut être effectué en utilisant un processus proche de celui utilisé dans les réactions de Schmidt et de Curtius, en effectuant la substitution de la fonction alcool par la fonction azide par addition des alcools bruts précédents à une suspension d'azidure de sodium dans l'acide trichloracétique, ou trifluoracétique et le chloroforme, à froid. Après traitement, on isole un mélange brut d'azides épimères que l'on utilise tel quel dans la suite.
c) Reduction des azides.
   Pour effectuer cette étape, on peut faire réagir le mélange précédent avec du nickel de Raney, dans de l'isopropanol, pour fournir après traitement un mélange d'amines primaires épimères utilisé tel quel dans la suite.
d) Formation du cycle pipéridinique et purification.
   Pour effectuer cette étape, on fait réagir dans l'acétonitrile (ou l'éthanol), à chaud, le dibromo-1,5 pentane éventuellement substitué par un ou plusieurs substituants choisis parmi les radicaux alkyle et les radicaux alkyle substitués par un atome d'halogène ou le groupe hydroxyle, sur le mélange précédent des amines primaires. Après traitement, on isole un résidu contenant essentiellement un mélange brut de deux épimères. On peut obtenir les deux isomères purs par chromatographie. Par barbotage de gaz chlorhydrique anhydre dans les solutions éthérées des amines ainsi séparées, on précipite les chlorhydrates hydrosolubles correspondants.

On peut préparer d'une façon analogue d'autres sels d'addition aux acides que le chlorhydrate comme précédemment.

En raison de leur action dopaminergique indirecte, les amines substituées de l'invention peuvent être utilisées dans des compositions pharmaceutiques, par exemple pour le traitement de la dépression et pour la stimulation de la vigilance.

En effet, comme on le verra plus loin, les amines substituées de l'invention ont la propriété d'avoir un effet stimulant non amphétaminique sur le système dopaminergique en stimulant également le système noradrénergique, ce qui permet de traiter la dépression nerveuse dans de bonnes conditions.

Aussi, l'invention a également pour objet des compositions pharmaceutiques comprenant au moins une amine substituée de formule (I) de l'invention, ou son sel d'addition à un acide pharmaceutiquement acceptable.

Les acides utilisables sont par exemple l'acide chlorhydrique, l'acide sulfurique et l'acide tartrique.

Pour la préparation des compositions pharmaceutiques, on peut dissoudre un sel d'addition aux acides des amines substituées conformes à l'invention dans une solution aqueuse injectable ou administrable par voie orale, par exemple du sérum physiologique.

On peut aussi inclure ces amines dans des préparations solides telles que des comprimés ou des gélules administrables par voie orale en utilisant des excipients et des techniques classiques.

Ces compositions pharmaceutiques peuvent comprendre des excipients, des diluants, des plastifiants et d'autres additifs pharmaceutiquement acceptables tels que ceux utilisés généralement dans les préparations pharmaceutiques. Les doses utilisées peuvent varier de 2,5 à 20mg/kg de poids corporel selon le mode d'administration et l'état du patient.

Pour l'administration par voie intramusculaire ou sous-cutanée, on peut utiliser des doses de 2,5 à 10mg/kg.

Pour l'administration par voie orale, on peut utiliser des doses de 10 à 20 mg/kg.

Lorsque la composition pharmaceutique est sous la forme d'une solution, la concentration de la solution en dérivé aminé est de préférence telle que le volume administré varie de 0,5 à 2ml.

Pour ces compositions pharmaceutiques, on peut utiliser les amines de l'invention sous la forme de leurs différents isomères ou d'un mélange d'isomères. Toutefois, comme on le verra ci-après, lorsque les amines substituées de l'invention comprennent un substituant alkyle R³ sur un noyau cyclohexyle, il est préférable d'utiliser l'isomère cis pour la position de R³ par rapport à l'atome d'azote du cycle pipéridine.

Dans le cas où l'amine substituée comprend un substituant alkyle R⁴ en position 2 d'un noyau cyclohexyle, il est préférable d'utiLiser également l'isomère cis pour la position de R⁴ par rapport à l'atome d'azote.

En revanche, dans le cas où l'amine substituée comprend un substituant alkyle R⁴ en position 3 du noyau cyclohexyle, il est préférable d'utiliser l'isomère trans.

D'autres caractéristiques et avantages de l'invention apparaîtront mieux à la lecture des exemples suivants.

Les exemples 1 à 8 illustrent la préparation d'alpha-aminonitriles utilisables pour préparer les amines substituées de l'invention des exemples 9 à 19 par le procédé en deux étapes.

L'exemple 20 illustre la préparation des deux formes isomères d'amine substituées conformes à l'invention par le procédé en 4 étapes.

Les exemples 21 à 23 et 25 illustrent les propriétés d'amines substituées conformes à l'invention dans des essais "in vitro", et

Les exemples 24 et 26 illustrent l'activité d'amines substituées de l'invention dans un essai "in vivo".

### Exemple 1 : Préparation du cyano-1 (méthyl-3 pipéridino)-1)-1 cyclohexane (synthon I).

On mélange 3,8g (0,039 mole) de cyclohexanone, 3,3g (0,039 mole) d'acétone cyanhydrine, 23,4g (0,19 mole) de HgSO₄ (séché) dans 5,77g (0,058 mole) de méthyl-3 pipéridine et 5g (0,058 mole) de diméthyl acétamide DMA. On agite fortement le mélange à 45° pendant 48h, puis on le verse dans un grand volume d'eau et de glace violemment agité pendant 30 min. Après extraction à l'éther, séchage sur (Na₂CO₃), évaporation du solvant sous vide, on obtient 7g (87,5%) du synthon I sous la forme d'une huile jaune de pureté supérieure à 95%, suffisante pour la suite des synthèses (IR : vers 2200cm⁻¹, GC/MS : 100-250°C (20°C/min), RT = 4,75min, m/e : 192,15.

### Exemple 2 : Préparation du cyano-1 ((éthylènekétal-4 pipéridino)-1)-1 cyclohexane (synthon II).

On mélange 3,2g (0,034 mole) de cyclohexanone, 2,85g (0,034 mole) d'acétone cyanhydrine, 20,2g (0,17 mole) de MgSO₄ séché dans 7,16g (0,05 mole)de dioxa-1,4 azaspiro-8 décane (4,5) et 4,4g (0,05 mole) de DMA. On agite fortement le mélange à 45° pendant 48h, puis on le verse dans un grand volume d'eau et de glace violemment agité pendant 30 min. Après extraction à l'éther, séchage sur Na₂CO₃, évaporation du solvant sous vide, on obtient 6g de synthon II sous la forme d'un résidu solide blanc (73,5%) qui fond à 108-109°C (IR vers 2200cm⁻¹

GC/MS : 100-250°C (20°C/min) RT = 8,32min, m/e 250,15).

### Exemple 3 : Préparation du cyano-1 ((hydroxyméthyl-3 pipéridino)-1)-1 cyclohexane (synthon III).

On mélange 8,83g (0,09 mole) de cyclohexanone, 7,66g (0,09 mole) d'acétone cyanhydrine, 32,4g (0,27 mole) de MgSO₄ (séché) avec 20,7g (0,18 mole)d'hydroxyméthyl-3 pipéridine et 10ml de DMA. On agite fortement le mélange à 45° pendant 48h, puis on le verse dans un grand volume d'eau et de glace violemment agité pendant 30 min. On extrait par 3x250ml d'éther, on sèche sur Na₂SO₄ et on évapore sous vide pour obtenir un résidu solide jaunâtre de 19g. Des cristallisations successives dans L'éthanol font précipiter 15g (38%) de synthon III sous la forme de cristaux incolores fondant à 94-95°C (IR vers 2200cm⁻¹, GC/MS : 100-250°C (20°C/min) RT = 8,14min, m/e 222,15).

### Exemple 4 : Préparation du cyano-1 ((diméthyl-3,5 pipéridino)-1)-1 cyclohexane (synthon IV).

On mélange 6g (0,06 mole) de cyclohexanone, 5,2g (0,06 mole) d'acétone cyanhydrine, 22g (0,18 mole) de MgSO₄ (séché) dans 27,6g (0,122 mole)de diméthyl-3,5 pipéridine et 11,3g (0,13 mole)de DMA. On agite fortement le mélange à 45° pendant 48h, puis on le verse dans un grand volume d'eau et de glace violemment agité pendant 30 min. Après extraction à l'éther, séchage sur Na₂CO₃, évaporation du solvant sous vide, on obtient 10g (76%) de synthon IV sous la forme d'un résidu solide blanchâtre ayant une pureté supérieure à 95% qui est suffisante pour la suite des synthèses (IR vers 2200cm⁻¹, GC/MS 100-250°C (20°C/min) RT = 5,44min, m/e 220,25).

### Exemple 5 : Préparation du cyano-1 (dipropylamino-1)-1 cyclohexane (synthon V).

On mélange 6,6g (0,067 mole) de cyclohexanone, 5,7g (0,067 mole) d'acétone cyanhydrine, 40,4g (0,34 mole) de MgSO₄) séché dans 10,2g (0,1 mole)de dipropylamine et 5ml de DMA. On agite fortement à 45° pendant 48h, puis on jette le mélange dans un grand volume d'eau et de glace violemment agité pendant 30 min. Après extraction à l'éther, séchage sur Na₂CO₃, évaporation du solvant sous vide, on obtient 10g (71,3%) de synthon V sous la forme d'une huile jaunâtre de pureté supérieure à 95%, suffisante pour la suite des synthèses (IR vers 2200cm⁻¹, GC/MS 100-250°C (20°C/min) RT = 4,60min, m/e 208,15).

### Exemple 6: Préparation du cyano-1 (diéthylamino-1)-1 cyclohexane (synthon VI).

On mélange 10,9g (0,11 mole) de cyclohexanone, 9,44g (0,11 mole) d'acétone cyanhydrine 40,4g (0,34 mole) de MgSO₄ séché dans 16,2g (0,22 mole) de diéthylamine et 9,6g de DMA. On agite fortement à 45° pendant 48h, puis on jette le mélange dans un grand volume d'eau et de glace violemment agité pendant 30 min. Après extraction à l'éther, séchage sur Na₂CO₃, évaporation du solvant sous vide, on obtient 15g (77,8%) du synthon VI sous la forme d'une huile jaunâtre de pureté supérieure à 95%, suffisante pour la suite des synthèses (IR vers 2200cm⁻¹, GC/MS 100-250°C (20°C/min) RT = 4,06min, m/e 180,20).

### Exemple 7 : Préparation du cyano-1 ((méthyl-4 pipéridino)-1)-1 cyclohexane (synthon VII).

On mélange 7,5g (0,078 mole) de cyclohexanone, 6,6g (0,078 mole) d'acétone cyanhydrine, 46,8g (0,38 mole) de MgSO₄ séché dans 11,5g (0,12 mole)de méthyl-4 pipéridine et 10g de DMA. On agite fortement à 45° pendant 48h, puis on jette le mélange dans un grand volume d'eau et de glace violemment agité pendant 30 min. Après extraction à l'éther, séchage sur (Na₂CO₃), évaporation du solvant sous vide, on obtient 13g (81,2%) du synthon VII sous La forme d'une huile jaunâtre de pureté supérieure à 95%, suffisante pour La suite des synthèses (IR vers 2200cm⁻¹, GC/MS).

### Exemple 8 : Préparation du cyano-1 ((diméthyl-3,5 pipéridino)-1)-1 méthyl-4 cyclohexane (synthon VIII).

On mélange 4,8g (0,04 mole) de méthyl-4 cyclohexanone, 3,6g (0,04 mole) d'acétone cyanhydrine, 15,4g (0,13 mole) de MgSO₄ séché dans 4,8g (0,04 mole) de diméthyl-3,5 pipéridine et 3,6g de DMA. On agite fortement à 45° pendant 48h, puis on jette le mélange dans un grand volume d'eau et de glace violemment agité pendant 30min. Après extraction à l'éther, séchage sur Na₂CO₃, évaporation du solvant sous vide, on obtient 7g (70%) de synthon VIII, sous la forme d'une huile jaunâtre de pureté supérieure à 95%, suffisante pour la suite des synthèses, contenant deux aminonitriles épimères qui, dans la suite (réaction de Bruylants), s'épimérisent vers le composé stable thermodynamiquement, seul à réagir (IR vers 2200cm⁻¹, GC/MS : 100-250°C (20°C/min)RT = 5,58min, RT = 5,62min, m/e 234,10.

### Exemple 9 : Préparation du (benzo(b)thiophényl-2)-1 ((méthyl-3 pipéridino)-1)-1 cyclohexane (composé n°1).

On prépare, dans 50ml d'éther anhydre, le réactif de Grignard résultant de l'action de 9,4g (0,036 mole) de iodo-2 benzo(b)thiophène sur 1,44g (0,06 mole) de magnésium en tournures. On y ajoute, lentement, 5g (0,024 mole) du synthon I de l'exemple 1 dissous dans 50ml d'éther anhydre. On agite 12h au reflux, on décompose le complexe par une solution saturée froide de NH₄Cl, puis, après décantation, on extrait les eaux à l'éther (3x50ml). Les phases éthérées réunies sont extraites par une solution aqueuse de HCl 20% (2x50ml). Les eaux acides sont neutralisées par NH₄OH, extraites à l'éther (3x50ml) ; les éthers rassemblés, après séchage sur (Na₂SO₄), sont évaporés sous vide pour donner un résidu solide jaune-brun de 6g. Ce dernier est chromatographie' sur alumine Merk (activité 2-3), dans un mélange d'éther de pétrole et d'éther (90/10 v/v) pour donner 4,9g (65%) du composé n° 1, solide blanc fondant à 85-86°C. Par barbotage de HCl gazeux dans la solution éthérée de ce composé, on fait précipiter son chlorhydrate, solide blanc qui, récupéré par essorage et séché sous vide, fond à 180-181°C (analytiquement pur) (GC/MS de la base ; 100-250°C (15°C/min) RT = 17,70min, m/e 313,2).Le spectre de RMN du ¹³C de ce composé et son analyse centésimale sont donnés dans les tableaux 2 et 3 annexés.

### Exemple 10 : Préparation du (benzo(b)thiophényl-2)-1 (dipropylamino-1)-1 cyclohexane (composé n°2).

On prépare, dans 50ml d'éther anhydre, le réactif de Grignard résultant de l'action de 13g (0,05 mole) de iodo-2 benzo(b)thiophène sur 2g (0,08 mole) de magnésium en tournures. On y ajoute, lentement, 7g (0,034 mole) du synthon V de l'exemple 5 dissous dans 50ml d'éther anhydre. On agite 16h au reflux, on décompose le complexe par une solution saturée froide de NH₄Cl, puis, après décantation, on extrait à l'éther (3x150ml). Les phases éthérées réunies sont extraites par une solution aqueuse de HCl 20% (2x200ml). Les eaux acides sont neutralisées par NH₄OH, extraites à l'éther (3x150ml) ; les éthers rassemblés, après séchage sur Na₂SO4, sont évaporés sous vide pour donner un résidu huileux jaunâtre de 8g. Ce dernier est chromatographie sur alumine Merck (activité 2-3) dans l'éther de pétrole, pour donner 7,4g (69%) de composé n° 2 sous La forme d'huile incolore. Par barbotage de HCl gazeux dans la solution éthérée de ce composé, on fait précipiter son chlorhydrate, solide blanc qui, récupéré par essorage et séché sous vide, fond à 157-158°C (analytiquement pur) (GC/MS de la base : 100-250°C (20°C/min) RT = 9,58 min, m/e 315,15).

Le spectre de RMN du ¹³C de ce composé et son analyse centésimale sont donnés dans les tableaux 2 et 3 annexés.

### Exemple 11 : Préparation du (benzo(b)thiophényl-2)-1 ((éthylénekétal-4 pipéridino)-1)-1 cyclohexane (composé n° 3.

On prépare, dans 50ml d'éther anhydre, le réactif de Grignard résultant de l'action de 7,3g (0,03 mole) de iodo-2 benzo(b)thiophène sur 1,2g (0,05 mole)) de magnésium en tournures. On y ajoute lentement 5g (0,02 mole) du synthon II de l'exemple 2 dissous dans 30ml d'éther anhydre. On agite 16h au reflux, on décompose le complexe par une solution saturée froide de NH₄Cl, puis, après décantation, on extrait Les eaux à l'éther (3x60ml). Les phases éthérées réunies sont Lavées à l'eau distillée (2x100ml) et, après séchage (Na₂SO₄), sont évaporées sous vide pour donner un résidu solide blanchâtre de 5g. Ce dernier est chromatographie sur alumine Merck (activité 2-3) dans L'éther de pétrole pour donner 4g de composé n° 3, solide blanc (56%) fondant à 80-81°C. Par barbotage de HCl gazeux dans la solution éthérée de ce composé, on fait précipiter son chlorhydrate, solide blanc qui, récupéré par essorage et séché sous vide, fond à 190-191°C (analytiquement pur) (GC/MS de la base : 100-250°C (20°C/min) RT = 19,94, m/e 357,25).

Le spectre de RMN du ¹³C de ce composé et son analyse centésimale sont donnés dans les tableaux 2 et 3 annexés.

### Exemple 12 : Préparation du (benzo(b)thiophényl-2)-1 ((diméthyl-3,5 pipéridino)-1)-1 cyclohexane (composé n° 4).

On prépare, dans 50ml d'éther anhydre, le réactif de Grignard résultant de l'action de 10,4g (0,04 mole) de iodo-2 benzo(b)thiophène sur 1,5g (0,06 mole)) de magnésium en tournures. On y ajoute lentement 4,5g (0,02 mole) du synthon IV de l'exemple 4 dissous dans 20ml d'éther anhydre. On agite 16h au reflux, on décompose le complexe par une solution saturée froide de NH₄Cl, puis, après décantation, on extrait à l'éther (3x50ml). Les phases éthérées réunies sont extraites par une solution aqueuse de HCl 20% (3x50ml). Les eaux acides sont neutralisées par NH₄OH et extraites à l'éther (2x70ml) ; les éthers rassemblés, après séchage sur Na₂SO₄ sont évaporés sous vide pour donner un résidu solide jaunâtre de 5g. Ce dernier est cristallisé deux fois dans le méthanol pour donner 4g du composé n° 4 sous la forme de cristaux blancs (61%) fondant à 98-99°C. Par barbotage de HCl gazeux dans la solution éthérée de ce composé, on fait précipiter son chlorhydrate, solide blanc qui, récupéré par essorage et séché sous vide, fond à 192-193°C (analytiquement pur) (GC/MS de la base : 70-250°C (15°C/min) RT = 17,62min, m/e 327,25).

Le spectre de RMN du ¹³C de ce composé et son analyse centésimale sont donnés dans Les tableaux 2 et 3 annexés.

### Exemple 13 : Préparation du (benzo(b)thiophényl-2)-1 ((méthyl-4 pipéridino)-1)-1 cyclohexane (composé n° 5).

On prépare, dans 70ml d'éther anhydre, le réactif de Grignard résultant de l'action de 10,4g (0,04 mole) de iodo-2 benzo(b)thiophène sur 1,5g (0,06 mole) de magnésium en tournures. On y ajoute lentement 4,3g (0,02 mole) du synthon VII de l'exemple 7 dissous dans 50ml d'éther anhydre. On agite 16h au reflux, on décompose le complexe par une solution saturée froide de NH₄Cl, puis, après décantation, on extrait à l'éther (3x100ml). Les phases éthérées réunies sont extraites par une solution aqueuse de HCl 20% (3x100ml).Les eaux acides sont neutralisées par NH₄OH, extraites à l'éther (2x100ml) et au chlorure de méthylène (2x100ml) ; les phases organiques rassemblées, après séchage sur Na₂SO₄ sont évaporées sous vide pour donner un résidu solide jaunâtre de 4g. Ce dernier est chromatographié sur alumine Merck (activité 2-3) dans un mélange d'éther et d'hexane (10/90 v/v) pour donner 3,7g du composé n° 5, solide blanc (59%) fondant à 84-85°C. Par barbotage de HCl gazeux dans la solution éthérée de ce composé, on fait précipiter son chlorhydrate, solide blanc qui, récupéré par essorage et séché sous vide, fond à 190-191°C (analytiquement pur) (GC/MS de la base : 70-250°C (15°C/min) RT = 18,52min, m/e 313,25).

Le spectre de RMN du ¹³C de ce composé et son analyse centésimale sont donnés dans les tableaux 2 et 3 annexés.

### Exemple 14 : Préparation du (benzo(b)thiophényl-2)-1 (diéthylamino-1)-1 cyclohexane (composé n° 6).

On prépare, dans 150ml d'éther anhydre, le réactif de Grignard résultant de l'action de 17,3g (0,066 mole) de iodo-2 benzo(b)thiophène sur 1,7g (0,07 mole) de magnésium en tournures. On y ajoute, lentement, 6g (0,033 mole) du synthon VI de l'exemple 6 dissous dans 50ml d'éther anhydre. On agite 16h au reflux, on décompose le complexe par une solution saturée froide de NH₄Cl, puis, après décantation, on extrait à l'éther (3x100ml). Les phases éthérées réunies sont extraites par une solution aqueuse de HCl 20% (3x100ml). Les eaux acides sont neutralisées par NH₄OH, extraites à l'éther (3x100ml) ; les éthers rassemblés, après séchage sur Na₂SO₄ sont évaporés sous vide pour donner un résidu solide jaunâtre de 6g. Ce dernier est chromatographie sur alumine Merck (activité 2-3) dans l'éther de pétrole pour donner 5,4g du composé n° 6 sous la forme d'huile incolore (58%). Par barbotage de HCl gazeux dans la solution éthérée de ce composé, on fait précipiter son chlorhydrate, solide blanc qui, récupéré par essorage et séché sous vide, fond à 160-161°C (analytiquement pur) (GC/MS de la base : 100-250°C (20°C/min) RT = 9,58min, m/e 287,15).

Le spectre de RMN du ¹³C de ce composé et son analyse centésimale sont donnés dans les tableaux 2 et 3 annexés.

### Exemple 15 : Préparation du (benzo(b)thiophényl-2)-1 ((hydroxyméthyl-3 pipéridino)-1)-1 cyclohexane (composé n° 7).

On prépare, dans 100ml d'éther anhydre, le réactif de Grignard résultant de l'action de 12,5g (0,048 mole) de iodo-2 benzo(b)thiophène sur 1,7g (0,07 mole) de magnésium en tournures. On y ajoute, lentement, 5,4g (0,024 mole) du synthon III de l'exemple 3 dissous dans 50ml d'éther anhydre. On agite 16h au reflux et on décompose le complexe par une solution saturée froide de NH₄Cl, puis, après décantation, on extrait les eaux à l'éther (3x100ml). Les phases éthérées réunies sont extraites par une solution aqueuse de HCl 20% (2x100ml). Les eaux acides sont neutralisées par NH₄OH 20%, extraites à l'éther (3x70ml) ; les éthers rassemblés, après séchage sur Na₂SO₄, sont évaporés sous vide pour donner un résidu huileux jaunâtre de 6g. Ce dernier est chromatographié sur alumine Merck (activité 2-3) dans un mélange d'éther et d'éther de pétrole (90/10 v/v), pour donner 5g (63%) de composé n° 7 sous la forme d'huile jaune claire. Celle-ci se tranforme lentement en cristaux incolores fondant à 102-103°C. Par barbotage de HCl gazeux dans la solution éthérée de ce composé on fait précipiter son chlorhydrate, solide blanc qui, récupéré par essorage et séché sous vide, fond à 188-189°C (analytiquement pur) (GC/MS de la base : 50-250°C (20°C/min) RT = 17,28min, m/e 329,15).

Le spectre de RMN du ¹³C de ce composé et son analyse centésimale sont donnés dans les tableaux 2 et 3 annexés.

### Exemple 16 : Préparation du (benzo(b)thiophényl-2)-1 ((bromométhyl-3 pipéridino)-1)-1 cyclohexane (composé n° 10).

A 5,7g (0,017 mole) du composé n° 7 de l'exemple 15 et 5ml de chlorure de méthylène, on ajoute très lentement (avec évacuation ou piégeage pour gaz acides), sous agitation, 3,6g (0,017 mole) de bromure de thionyle dissous dans 2ml de chlorure de méthylène. On agite une nuit à température ambiante puis on évapore le solvant sous vide. Le résidu obtenu est repris dans l'éther, lavé avec HCl 10% (2x200ml) . La phase aqueuse est neutralisée par NH₄OH 20%, extraite à l'éther (2x70ml), puis au chlorure de méthylène (70ml). Après réunion et séchage sur Na₂SO₄, les phases organiques sont évaporées sous vide pour fournir un résidu huileux brunâtre de 3g. Celui-ci est purifié par flash chromatographie sur silice, dans l'éther de pétrole, pour donner 2,7g (41%) du composé n°8 sous la forme d'huile incolore. Par barbotage de HCl gazeux dans la solution éthérée de la base, on fait précipiter son chlorhydrate, solide blanc qui, récupéré par essorage et séché sous vide, fond à 178-179°C (analytiquement pur) (GC/MS de la base : 100-250°C (20°C/min) RT = 16,42min, m/e 391,5 et 393,05).

Le spectre de RMN du ¹³C de ce composé et son analyse centésimale sont donnés dans les tableaux 2 et 3 annexés.

### Exemple 17 : Préparation du benzo(b)thiophényl-2)-1 ((iodométhyl-3 pipéridino)-1)-1 cyclohexane (composé n° 9).

A 2g (0,006 mole) du composé n°7 de l'exemple 15 et 10ml de chlorure de méthylène, on ajoute lentement sous agitation et en atmosphère d'azote, 4,86g (0,024 mole) d'iodure de triméthylsilane. Le mélange est porté, sous agitation, à 40°C pendant 24h, refroidi et jeté dans une solution froide de bisulfite de sodium puis extrait au chlorure de méthylène (3x30ml). La phase organique est séchée sur Na₂SO₄ et évaporée sous vide pour donner 1,2g de résidu huileux brunâtre. Celui-ci est purifié par chromatographie sur alumine Merck (activité 2-3) dans un mélange d'éther de pétrole et d'éther (90/10 v/v) pour donner 0,7g (18%) du composé n° 9 sous la forme d'huile claire. Par barbotage de HCl gazeux dans la solution éthérée de ce composé, on fait précipiter son chlorhydrate, solide blanc qui, récupéré par essorage et séché sous vide, fond à 155-156°C (analytiquement pur) (GC/MS de la base : 100-250°C (20°C/min) RT = 19,07min, m/e 439).

Le spectre de RMN du ¹³C de ce composé et son analyse centésimale sont donnés dans les tableaux 2 et 3 annexés.

### Exemple 18 : Préparation du (benzo(b)thiophényl-2)-1 ((chlorométhyl-3 pipéridino)-1)-1 cyclohexane (composé n° 10).

A 3g (0,009 mole) du composé n° 7 de l'exemple 15, et 5ml de chlorure de méthylène, on ajoute très lentement (avec évacuation ou piégeage pour gaz acides), sous agitation, 1,9g (0,009 mole) de chlorure de thionyle dissous dans 2ml de chlorure de méthylène. On agite 6h à 60°C puis après refroidissement on jette le milieu dans une solution de Na₂CO₃. Après l5min d'agitation, on extrait au chlorure de méthylène (3x20ml), on sèche sur Na₂SO₄, puis on évapore le solvant sous vide pour obtenir un résidu huileux brunâtre de 2g. Celui-ci est purifié par flash chromatographie sur silice, dans l'éther de pétrole et l'éther (90/10 v/v), pour donner 1g (32%) de composé n° 10 sous la forme d'huile incolore. Par barbotage de HCl gazeux dans la solution éthérée de ce composé, on fait précipiter son chlorhydrate, solide blanc qui, récupéré par essorage et séché sous vide, fond à 182-183°C (analytiquement pur) (GC/MS de la base : 100-250°C (20°C/min) RT = 29min, m/e 347,25).

Le spectre de RMN du ¹³C de ce composé et son analyse centésimale sont donnés dans les tableaux 2 et 3 annexés.

### Exemple 19 : Préparation du (benzo(b)thiophényl-2)-1-r-((diméthyl-3,5 pipéridino)-1)-1 c-méthyl-4 cyclohexane (composé n° 11).

On prépare, dans 150ml d'éther anhydre, le réactif de Grignard résultant de l'action de 15g (0,06 mole) de iodo-2 benzo(b)thiophène sur 1,7g (0,07 mole) de magnésium en tournures. On y ajoute lentement 6,75g (0,03 mole) du synthon VIII de l'exemple 8 dissous dans 50ml d'éther anhydre. On agite 16h au reflux, on décompose le complexe par une solution saturée froide de NH₄Cl, puis, après décantation, on extrait à l'éther (3x100ml). Les phases éthérées réunies sont extraites par une solution aqueuse de HCl 20% (3x100ml). Les eaux acides sont neutralisées par NH₄OH, extraites à l'éther (2x70ml), puis au chlorure de méthylène (2x70ml) ; les phases organiques rassemblées, après séchage sur Na₂SO₄, sont évaporées sous vide pour donner un résidu huileux de 6g. Ce dernier donne, après flash chromatographie sur silice dans l'éther de pétrole, 5,4g (53%) du composé n° 11, solide blanc fondant à 115-116°C. Par barbotage de HCl gazeux dans la solution éthérée de ce composé, on fait précipiter son chlorhydrate, solide blanc qui, récupéré par essorage et séché sous vide, fond à 198-199°C (analytiquement pur) (GC/MS de la base : 100-250°C (20°C/min) RT = 10,90min, m/e 341,15).

Le spectre de RMN du ¹³C de ce composé et son analyse centésimale sont donnés dans les tableaux 2 et 3 annexés.

### Exemple 20 : Préparation du (benzo(b)thiophényl-2)-1 t-méthyl-4-r-(pipéridino-1)-1 cyclohexane (composé n°12) et du (benzo(b)thiophényl-2)-1 c-méthyl-4-r-(pipéridino-1)-1 cyclohexane (composé n°13).

A) On prépare, dans 150ml d'éther anhydre, le réactif de Grignard résultant de l'action de 29g (0,112 mole) de iodo-2 benzo(b)thiophène sur 3,1g (0,13 mole) de magnésium en tournures. On y ajoute 9,1g (0,081 mole) de méthyl-4 cyclohexanone dissous dans 150ml d'éther anhydre. On agite 12h au reflux, on décompose le complexe par une solution saturée froide de NH₄Cl, puis, après décantation, on extrait les eaux à l'éther (2x150ml) puis au chlorure de méthylène (2x150ml). Les phases organiques, séchées sur Na₂SO₄ sont évaporées sous vide pour donner 17g d'une huile jaune pâle contenant essentiellement deux alcools épimères (IR, GC/MS : 100-250°C (20°C/min)RT = 14,28min, m/e 246,10 ; RT = 14,50min, m/e 246,10). La réaction suivante (B) procédant par carbocation, les alcools ne sont pas autrement purifiés.
B) On prépare à -20°C une suspension contenant 7,9g (0,12 mole) d'azidure de sodium, 69,5g (0,6 mole) d'acide trichloracétique et 250ml de chloroforme et on agite fortement. On y ajoute, rapidement, dissous dans 150ml de chloroforme et à la même température, 15g des alcools bruts obtenus précédemment. L'agitation et la température sont maintenues pendant 3h (ou jusqu'à disparition des alcools). Le milieu pâteux obtenu est versé dans une solution froide de Na₂CO₃. Après décantation, on extrait au chloroforme (2x100ml) et on Lave les phases organiques rassemblées jusqu'à pH neutre. Après séchage sur Na₂SO₄, évaporation sous vide, on récupère un résidu huileux pesant 16g, contenant essentiellement un dérivé insaturé (très minoritaire) et deux azides épimères (IR) qui, compte tenu de leur relative instabilité, ne sont pas autrement purifiés.
C) 15g du mélange des deux azides précédemment obtenus sont dissous dans 150ml d'isopropanol et chauffés à 65° pendant 30min. On ajoute, par portions, du nickel de Raney (en maintenant la température)jusqu'à cessation de dégagement gazeux. On chauffe alors à 70° pendant 15min, on refroidit à la température ambiante et on filtre sur colite. Le filtrat, dilué au chlorure de méthylène, est lavé à l'eau, séché sur Na₂CO₃ et évaporé sous vide et donne finalement un résidu huileux. Celui-ci, dissous dans HCl 10%, est lavé à l'éther (2x200ml), la phase aqueuse est neutralisée par NH₄OH 20% et extraite l'éther (2x200ml). Après séchage (Na₂CO₃), évaporation sous vide, on recueille un résidu huileux pesant 9g qui contient essentiellement deux amines primaires épimères (IR).
D) 7g du mélange d'amines précédent sont dissous dans 100ml d'acétonitrile contenant 6,6g de dibromo-1,5 pentane (0,028 mole) et 7,9g (0,057 mole) de K₂CO₃. Le mélange, fortement agité, est porté au reflux pendant 48h puis refroidi à la température ambiante. Après filtration, on ajoute 100ml d'HCl 20%, on extrait à l'éther (2x50ml). Les eaux acides, neutralisées par NH₄OH, sont à leur tour extraites à l'éther (3x50ml). Après séchage sur Na₂CO₃, ces éthers sont évaporés sous vide pour donner un résidu huileux rouge de 7,1g. Celui-ci est chromatographie sur alumine Merck (activité 2-3). L'éther de pétrole élue une première fraction de 4g de cristaux blancs du composé n° 13 fondant à 113-114°C et un mélange d'éther de pétrole-éther (50/50 v/v) élue une deuxième fraction de 2,3g de cristaux blancs du composé n° 12 fondant à 120-121°C (38,6% rdt global à partir de la cétone). Par barbotage de HCl gazeux dans la solution éthérée des composés, on fait précipiter leurs chlorhydrates, solides blancs qui, récupérés par essorage et séchés sous vide, fondent respectivement à 195-196°C (n° 13) et 209-210°C (n° 12) (analytiquement purs) (GC/MS des bases : 100-250°C (20°C/min), n° 20 : RT = 10,56min, m/e 313,15 ; n° 21 : RT = 10,86min, m/e 313,15).

Le spectre de RMN du ¹³C de ces composés et leur analyse centésimale sont donnés dans les tableaux 2 et 3 annexés.

### Exemple 21 : Test d'inhibition de la capture de (³H)dopamine par les synaptosomes striataux de rat.

Ce test est pratiqué selon la méthode utilisée par Vignon et Lazdunski en opérant de la façon suivante. Des rats Wistar pesant 200-250g sont tués par dislocation cervicale. Leurs cerveaux sont rapidement prélevés et les striata sont disséqués sur la glace. Les striata sont ensuite homogénéisés (10 allers-retours) avec un homogénéisant Potter dans 50 volumes de tampon Tris HCl 10 mM à pH 7,4 contenant 0,32 M de sucrose. L'homogénat est ensuite centrifugé à 1000g pendant 10min à 4°C, puis le surnageant obtenu est recentrifugé à 10000g pendant 20min à 4°C. Les culots résultants P2 contenant les synaptosomes bruts sont ensuite utilisés sans purification supplémentaire.

Les culots sont repris dans un milieu de Ringer (mM : 140 NaCl, 5 KCl, 2,6 CaCl₂, 1,3 MgSO₄ 10 Tris-HCl, pH = 7,4) + 200 µM de pargyline et équilibrés pendant 30 min à 30°C. Le composé à tester est préincubé à la concentration désirée pendant 30 min à 30°C en présence du culot P2 à une concentration finale en protéine de 0,15-0,20 mg/ml dans le milieu de Ringer.

On ajoute alors de la dopamine tritiée ((³H)DA, Amersham) à une concentration finale de 5 nM pendant 5 min à 30°C, et on interrompt sa capture par filtration de 150 µl du milieu d'incubation sur des filtres en fibres de verre GF/B (Whatman). La radioactivité retenue par les filtres est mesurée dans un compteur à scintillation liquide (LKB)avec 3 ml d'ACS (Amersham) comme scintillant. La capture non spécifique est mesurée à 4°C dans des incubations parallèles : elle est ensuite soustraite de la capture totale mesurée à 30°C. L'IC50 est la concentration en composé qui inhibe 50% de la capture spécifique obtenue en absence d'inhibiteur.

Les composés à tester sont solubilisés au départ dans de l'eau à une concentration de 1mM et les autres concentrations sont obtenues par des dilutions successives de cette solution mère dans l'eau.

Les résultats obtenus sont donnés dans le tableau 4 annexé.

On constate au vu de ce tableau que les composés testés sont capables, à très faible concentration, d'inhiber la capture synaptosomale de dopamine.

### Exemple 22 : Affinité des composés pour les sites de liaison de la (³H)BTCP sur les membranes striatales de cerveaux de rats.

Les striata de cerveaux de rats rapidement disséqués dans la glace sont homogénéisés avec un ultra turax pendant 30 s (position 6) dans 50 volumes d'une solution de sucrose 0,32 M tamponnée par du Tris-HCl 10 mM à pH = 7,4. L'homogénat est centrifugé à 4°C à 1000g pendant 10 min, puis le surnageant obtenu est centrifugé à 40000 g à 4°C pendant 30 min et les culots obtenus sont repris dans 1 ml de tampon par striatum.

L'affinité des composés pour le complexe de recapture de la dopamine est déterminée par des expériences de compétition sur les membranes de striatum préparées comme ci-dessus. Le ligand radioactif utilisé est la (³H)BTCP (Service des Molécules Marquées du CEA, Saclay France) à 55Ci/mmol.

Les membranes (concentration finale de protéine 0,1 à 0,2 mg/ml) sont incubées dans 1 ml final de tampon phosphate de sodium 50 mM, pH = 7,4 en présence d'une concentration fixe de ligand tritié (0,2 nM) et de concentrations croissantes du composé à tester. Après 90 min à 4°C, 3 aliquots de 250 µl sont filtrés sous vide sur des filtres de fibre de verre GF/B (Whatman). La liaison non spécifique est obtenue dans des expériences parallèles réalisées en présence de 10 µM de BTCP non marquée. Elle est soustraite de la liaison totale pour obtenir la liaison spécifique. La concentration de composé qui empêche 50% de la liaison spécifique du ligand radioactif (IC₅₀) reflète l'affinité de ce dernier pour le complexe de recapture de la dopamine dans les terminaisons nerveuses.

Les résultats obtenus sont donnés dans le tableau 4 ci-joint.

Au vu de ces résultats, il apparaît que les composés de l'invention sont très actifs comme inhibiteurs de la recapture de la dopamine par les terminaisons nerveuses puisqu'ils inhibent de la même façon à des faibles concentrations (IC₅₀ de l'ordre du nanomolaire) à la fois la recapture de la dopamine et la fixation de la (³H)BTCP. Il existe une excellente corrélation entre l'affinité des composés pour le site (³H)BTCP et leur pouvoir inhibiteur de la recapture de la (³H)DA(R=0,99 ; n = 13 ; p < 0,001). A titre comparatif nous avons porté dans ce tableau les résultats obtenus avec la nomifensine (inhibiteur connu de la recapture de la DA) ; celle-ci est beaucoup moins puissante dans cet effet que la plupart des composés testés.

### Exemple 23 : Affinité des composés pour les sites de liaison de la (³H)TCP sur les homogénats de cerveaux de rats.

Dans cet essai on mesure les affinités des composés pour le récepteur de la PCP par des expériences de compétition sur des homogénats de cerveaux de rats. Le ligand radioactif utilisé est la (³H)TCP à une activité spécifique de 62 Ci/mmol (Service des Molécules Marquées, CEA Saclay France).

Les cerveaux de rats (sans le cervelet et le tronc cérébral) sont homogénéisés à l'ultra turax dans 20 volumes de tampon Hepes-Tris 50 mM, pH 7,7. Après centrifugation pendant 30 min à 15000 g le culot est repris dans le même volume de tampon et recentrifugé dans les mêmes conditions. Le culot final est repris dans du tampon Hepes-Tris 50 mM, pH 7,7 de façon à obtenir une concentration en protéine de 6 à 8 mg/ml.

Les expériences de compétition sont réalisées en incubant pendant 30 min les membranes (concentration finale en protéine : 0,6 à 0,8 mg/ml) en milieu tampon 5 mM Hepes-Tris, pH 7,7 à 25°C, en présence d'une concentration fixe de ligand tritié (1 nM)et de concentrations croissantes de composés à tester. Trois échantillons de 600 µL sont ensuite filtrés sur filtres de fibres de verre GF/B prétraités par du PEI (polyéthylénimine) à 0,1%. Les filtres sont rincés avec 3x5ml de tampon Tris-HCl 10 mM, NaCl 100 mM et la radioactivité retenue est mesurée dans un compteur à scintillation liquide. La liaison non spécifique est obtenue en présence de TCP 100 µM. L'IC50 est la concentration du composé qui inhibe 50% de la liaison spécifique du ligand radioactif.

Les résultats obtenus sont donnés dans le tableau 4 annexé.

On peut constater que tous les composés testés ont une très faible affinité pour le récepteur de la PCP (ou pas d'affinité) et doivent par conséquent présenter peu d'activité psychotomimétique liée à l'intéraction des molécules avec ce récepteur. D'autre part, il existe un facteur très important de sélectivité pour le complexe de recapture de la dopamine comparé au récepteur de la PCP. Ainsi les composés n° 13 et 2 donnent de très bons résultats.

### Exemple 24: Mesure de la capture de noradrénaline.

La capture de noradrénaline tritiée (40 Ci/mmole) est évaluée à partir de préparations synaptosomales brutes d'hypothalamus de rats (Sprague-Dawley mâle, 200-300g, Charles River, St Aubin lès Elbeuf, France).

Les animaux sont sacrifies par décapitation et les cerveaux sont rapidement dégagés. Les hypothalami, disséqués à 0-4°C, sont homogénéisés dans 10 volumes (poids/volume) de saccharose 0,32 M préalablement refroidi, au moyen d'un homogénéiseur type Potter-Elvejhem (clairance 80-130 µm, 800 rpm). Les débris cellulaires et le matériel nucléaire sont éliminés par centrifugation (1000 g, 10 min, 4°C). Le surnageant constitue la fraction synaptosomale brute.

Des aliquots (50 µl) de préparation synaptosomale sont préincubés pendant 5 minutes à 37°C, en présence de pargyline 20 µM, et de concentrations croissantes des composés à tester, dans 960 µl d'un milieu Krebs-Ringer enrichi en glucose (composition en mM : NaCl 103, CaCl₂1, MgCl₂ 1, KH₂PO₄ 1, NaHCO₃ 27, acide ascorbique 0,1, glucose 5,4). A l'issue de cette préincubation, la noradrénaline tritiée (concentration finale 50 nM) est ajoutée sous un volume de 40 µl, et l'incubation est poursuivie à 37°C pendant 10 minutes. La réaction est arrêtée par dilution avec 2 ml de milieu glacé et centrifugation (7000 g, 10 minutes, 4°C). Après lavage du culot de centrifugation dans 1 ml de milieu glacé, l'échantillon est à nouveau centrifugé dans les mêmes conditions. Le culot obtenu est remis en suspension dans 250 µl d'eau distillée par sonication (Sonotrode TC 4C). 100 µl de cette suspension sont utilisés pour la mesure de la radioactivité par scintillation liquide (SL 2000, Kontron intertechniques, Trappes, France) et 50 µl pour la détermination des taux de protéines (selon la méthode de Lowry et al.).

La capture non spécifique est étudiée simultanément à 0°C en présence des différentes concentrations de composé.

La capture spécifique (capture totale - capture à 0°C) est exprimée en fentomoles/mg de protéines.

Pour chaque concentration étudiée la mesure était faite en double, 3 à 5 expériences étant pratiquées à des jours différents (préparations synaptosomales différentes). Pour chaque composé, au moins 4 concentrations ont été étudiées (entre 10⁻⁹ et 10⁻⁶M habituellement).

Les résultats sont donnés dans le tableau 5 annexé.

### Exemple 25 : Mesure de l'activité locomotrice.

L'activité locomotrice de souris Swiss (mâles CD1, 25-30 g, Charles River, St Aubin lès Elbeuf, France) est mesurée dans l'actimètre Boissier-Simon (Apelex, Bagneux, France).

30 minutes après l'administration des composés à tester (10 mg/kg, i.p.), les souris sont introduites dans des cages individuelles en Plexiglass (L = 26 cm, l = 20 cm, h = 10 cm), équipées de deux cellules photoélectriques situées à 1 cm au-dessus du plancher. Chaque cellule est reliée à un compteur électromécanique. "L'activité locomotrice" correspond au nombre de rayons traversés, de la 20ème à la 35ème minute après introduction dans les cages d'actimétrie.

Les résultats sont donnés dans le tableau 5 annexé.

Au vu de ces résultats, on constate que les composés n° 1 et 13 ont un effet supérieur à celui du BTCP.

Dans les tableaux 4 et 5, on a donné à titre comparatif les résultats obtenus avec le BTCP, la Nomifensine et le Halopéridol dans les essais des exemples 33 à 36.

Le BTCP (benzo(b)thiophényl-2)-1 (pipéridino-1 cyclohexane a été préparé de la façon suivante :

A un réactif de Grignard, préparé à partir de 31,2g (0,12 mole) d'iodo-2 benzo(b)thiophène et 2,8g de magnésium en tournures dans 100ml d'éther anhydre, on ajoute goutte à goutte, à température ambiante, 12g (0,063 mole) dans 200ml d'éther anhydre, du synthon I de l'exemple 1. La solution est portée au reflux pendant 16h, puis refroidie et jetée dans une solution saturée de NH₄Cl et de glace. Après 30 min d'agitation, puis décantation, on extrait à l'éther (3x200ml), on lave les éthers par HCl 10% (3x200ml). Les eaux acides sont neutralisées par NH40H 20%, extraites à l'éther (3x200ml) et la phase organique lavée à l'eau jusqu'à pH neutre. Après séchage (Na₂SO₄) et évaporation sous pression réduite, on obtient un résidu solide blanc de 15,6g. Celui-ci est cristallisé deux fois dans l'éthanol pour donner 14g de cristaux incolores (75% fondant à 80-81°C. Par barbotage de HCl gazeux dans la solution éthérée de la base, on fait précipiter son chlorhydrate, solide blanc qui, récupéré par essorage et séché sous vide, fond à 194-195°C (analytiquement pur) (GC/MS de la base : 100-250°C (20°C/min) RT = 10,36 min, m/e 299,20).

La Nomifensine, soit l'amino-8 méthyl-2 phényl-4 tétrahydro- 1,2,3,4 isoquinoline, est un produit connu pour agir sur le système de recapture de la dopamine.

L'Halopéridol est un antagoniste dopaminergique n'agissant pas sur la recapture.

Les résultats des tableaux 3 et 4 montrent que les amines de l'invention ont un effet plus important que la Nomifensine et que leur effet n'est pas du même type que celui de l'Halopéridol.

Par ailleurs, elles ont une sélectivité pour le complexe de recapture de la dopamine meilleure que celle du BTCP.

Le tableau 6 annexé illustre les résultats d'essais de toxicité effectués chez la souris sur les amines substituées de l'invention à une dose de 100mg/kg par voie intrapéritonéale.

### Exemple 26 : Etude de l'inhibition de la recapture de la dopamine et de la noradrénaline sur des neurones en culture.

a) Cultures primaires de neurones.
   La substance noire, structure riche en cellules dopaminergiques, ou le locus coeruleus, contenant les corps cellulaires noradrénergiques, sont disséqués à partir d'embryons de rats de 14 jours, âge auquel les cellules ont terminé leur mitose. Les cellules sont dissociées mécaniquement dans un milieu sans calcium ni magnésium.
   L'ensemencement est réalisé dans des puits prétraités avec de la D-polylysine, à une densité de 150 000 cellules/puits, dans 400µl de milieu de culture composé de 70% de M.E.M. ; 25% de Hanks ; 5% de Nu-serum décomplémenté ; plus 3% de D-glucose (1/20).
   Le milieu de culture est renouvelé au tiers tous les trois jours, jusqu'à 9 jours de développement "in vitro", âge auquel les cellules paraissent matures. A cet âge, la culture ne présente pas encore de cellule gliales.
b) Test d'inhibition de la recapture de la dopamine et de la noradrénaline.

On pré-incube les cellules obtenues en a) pendand 15min, à la température ambiante, avec 400µl d'un tampon constitué par le Ringer Phosphatedisodique 5 mM pH=7,6 dont la composition en concentrations finales est la suivante : NaCl 120mM ; CaCl₂ 2,6 mM ; MgCl₂ 1,3 mM ; KCl 5mM ; D-glucose 3 mM ; Na₂HPO₄5mM.

On incube ensuite pendant 10min à 37°C avec le même tampon en présence de (³H)Da ou de (³H)NA à une concentration finale 10⁻⁸M et une dilution isotopique de 1/5, et de concentrations croissantes en composé à tester dans un volume final de 400µl.

On rince ensuite trois fois rapidement avec 750µl du même tampon à 4°C, ce qui correspond à une durée totale de 10 secondes.

On récupère alors les cellules dans 2 fois 500µl de NaOH 0,5N et on détermine leur radioactivité par comptage avec 4ml de scintillant et 50µl d'acide acétique glacial.

Avant les rinçages, on a prélevé un aliquote de 100µl du milieu d'incubation et on a déterminé la radioactivité libre sur celui-ci par comptage avec 5ml de scintillant.

On évalue également la diffusion passive de (³H)DA ou de (³H)NA dans les cellules en présence de 10⁻⁴M/l de Nomifensine ou de Désipramine qui sont respectivement des inhibiteurs de recapture de la dopamine et de la noradrénaline en suivant le même mode opératoire sans composé à tester.

La recapture spécifique de la (³H)DA ou de (³H)NA est déduite de la différence entre la recapture totale et la diffusion passive pour chacun des composés testés.

Les résultats obtenus sont donnés dans le tableau 7 annexé.

Au vu de ces résultats, on constate que le composé n° 4 apparaît comme un inhibiteur très puissant de la recapture de la dopamine mais aussi de la noradrénaline.

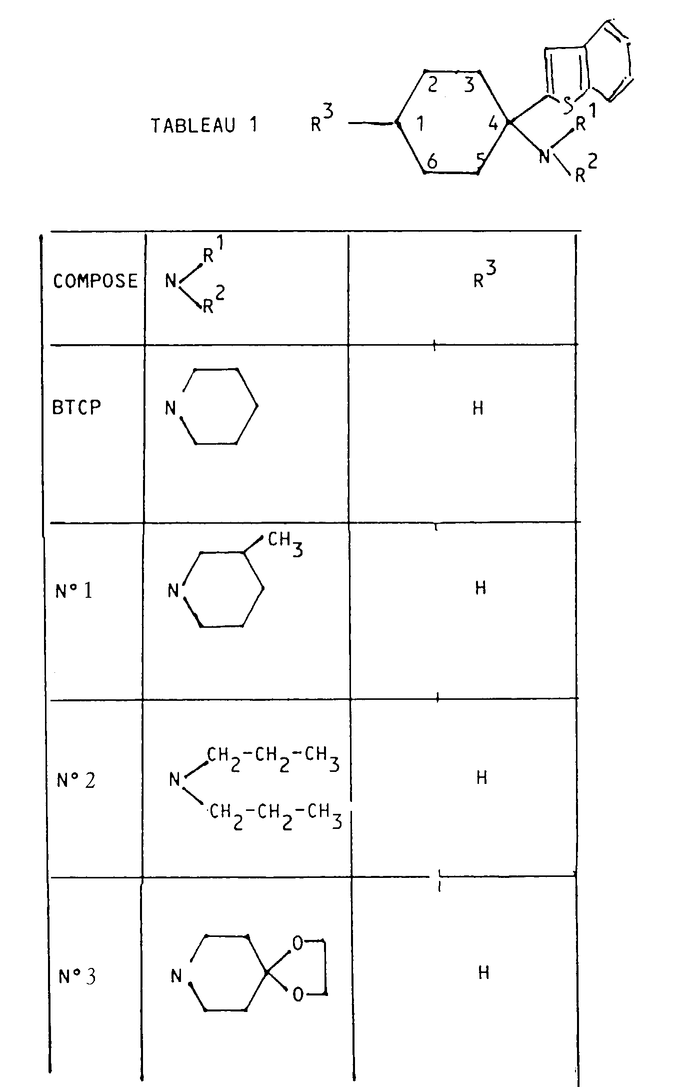

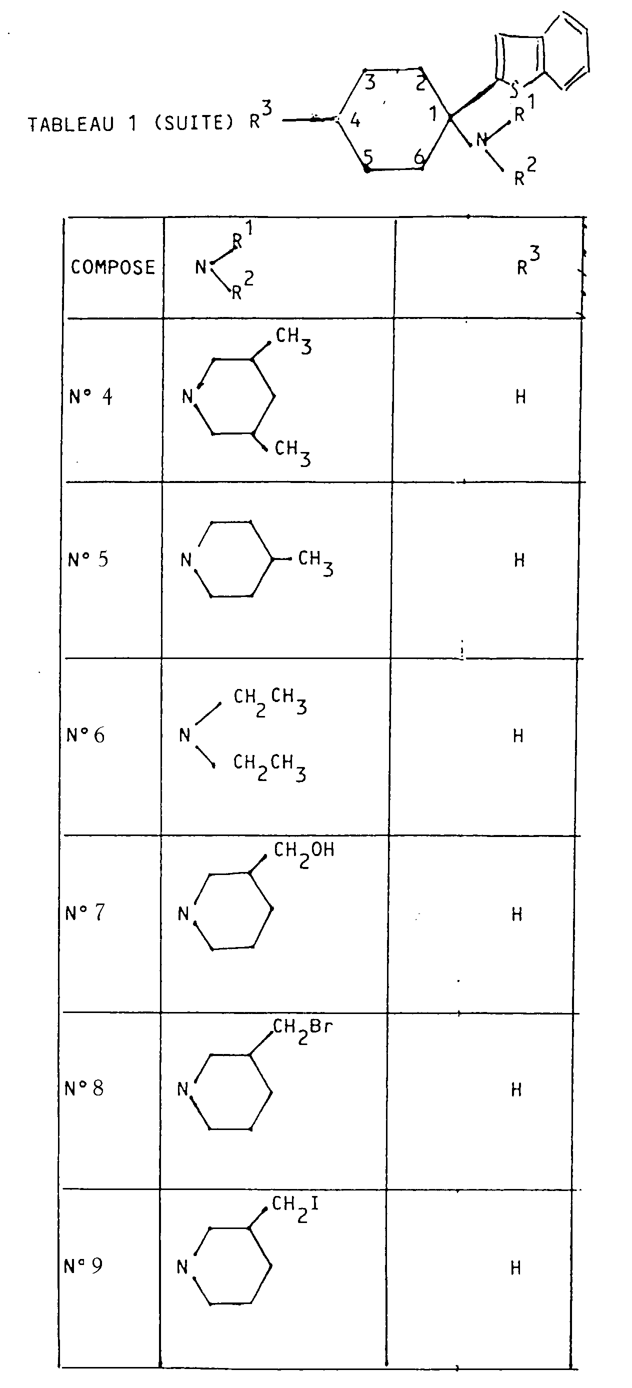

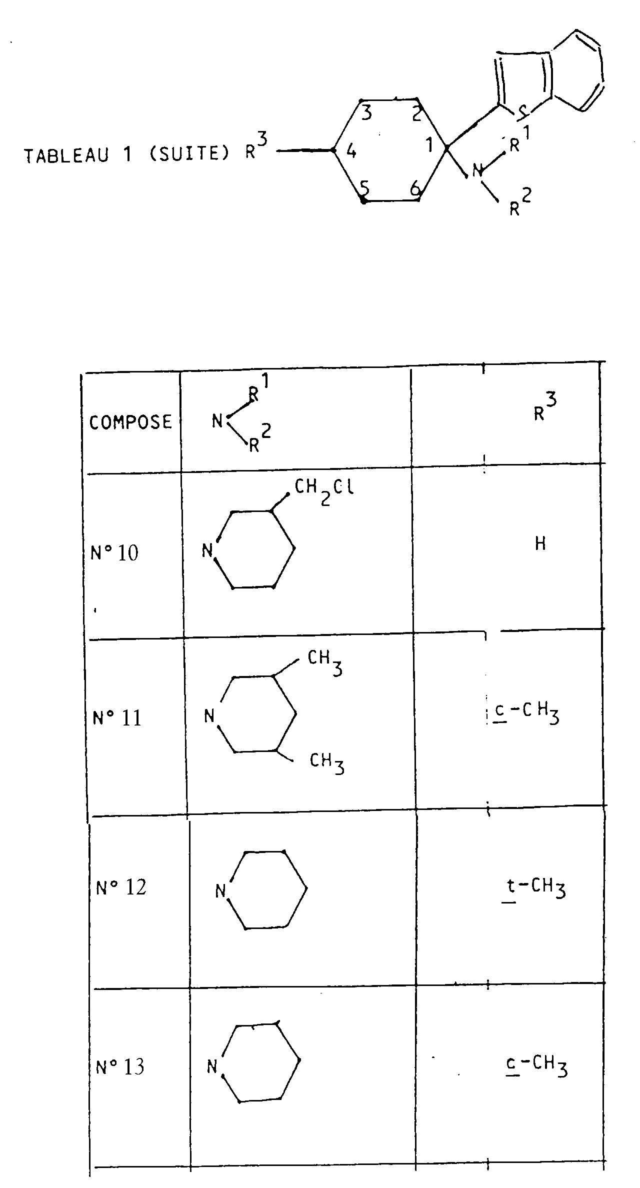

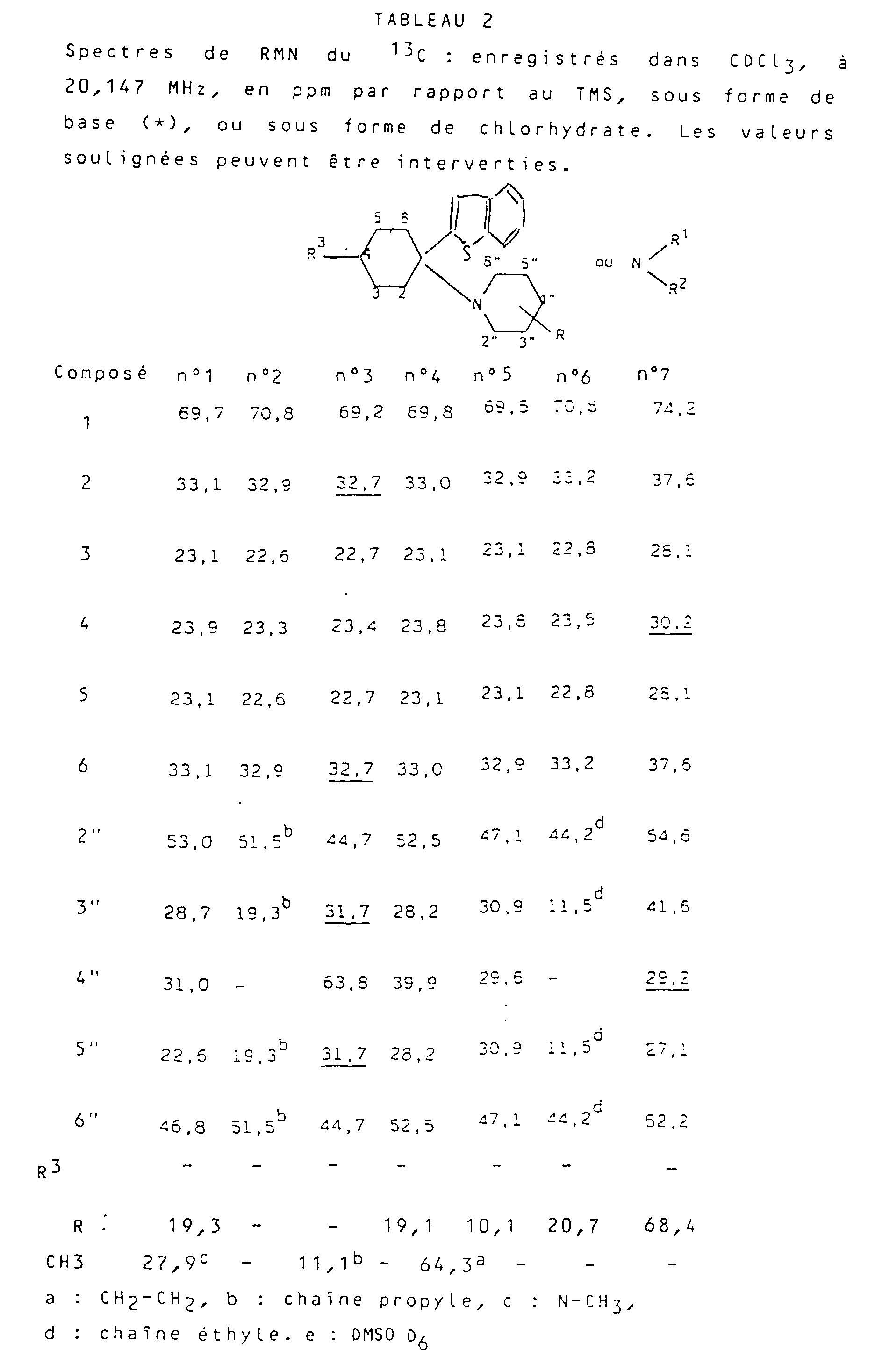

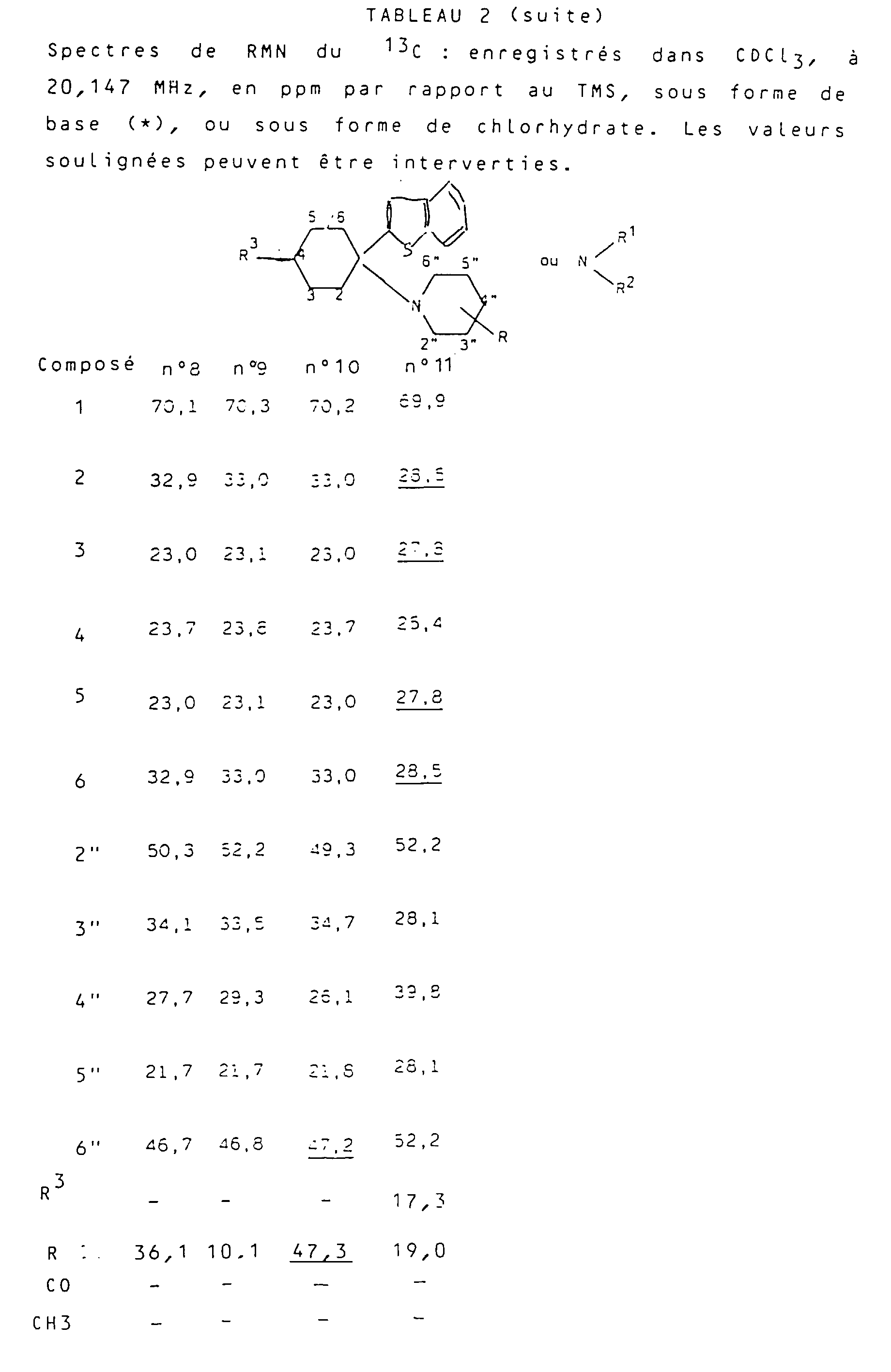

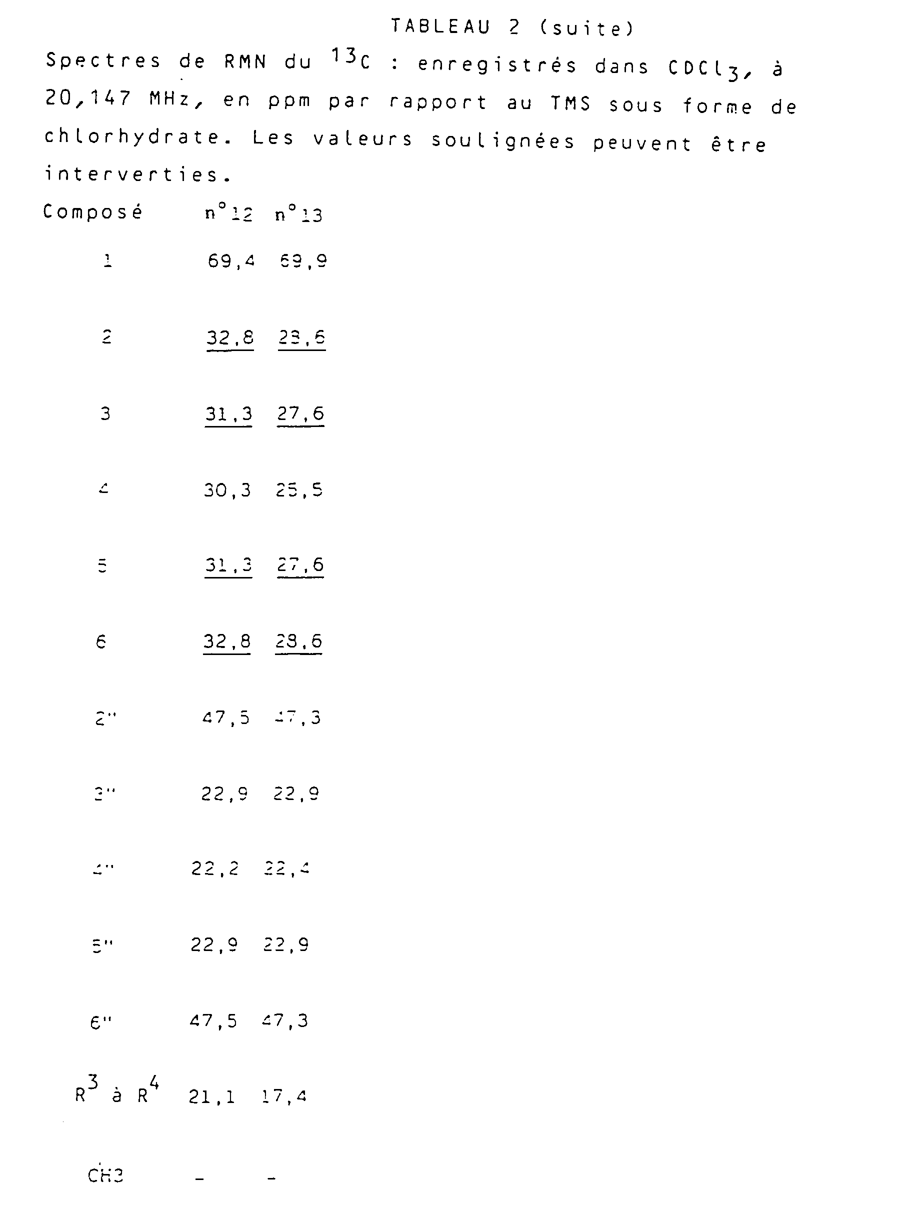

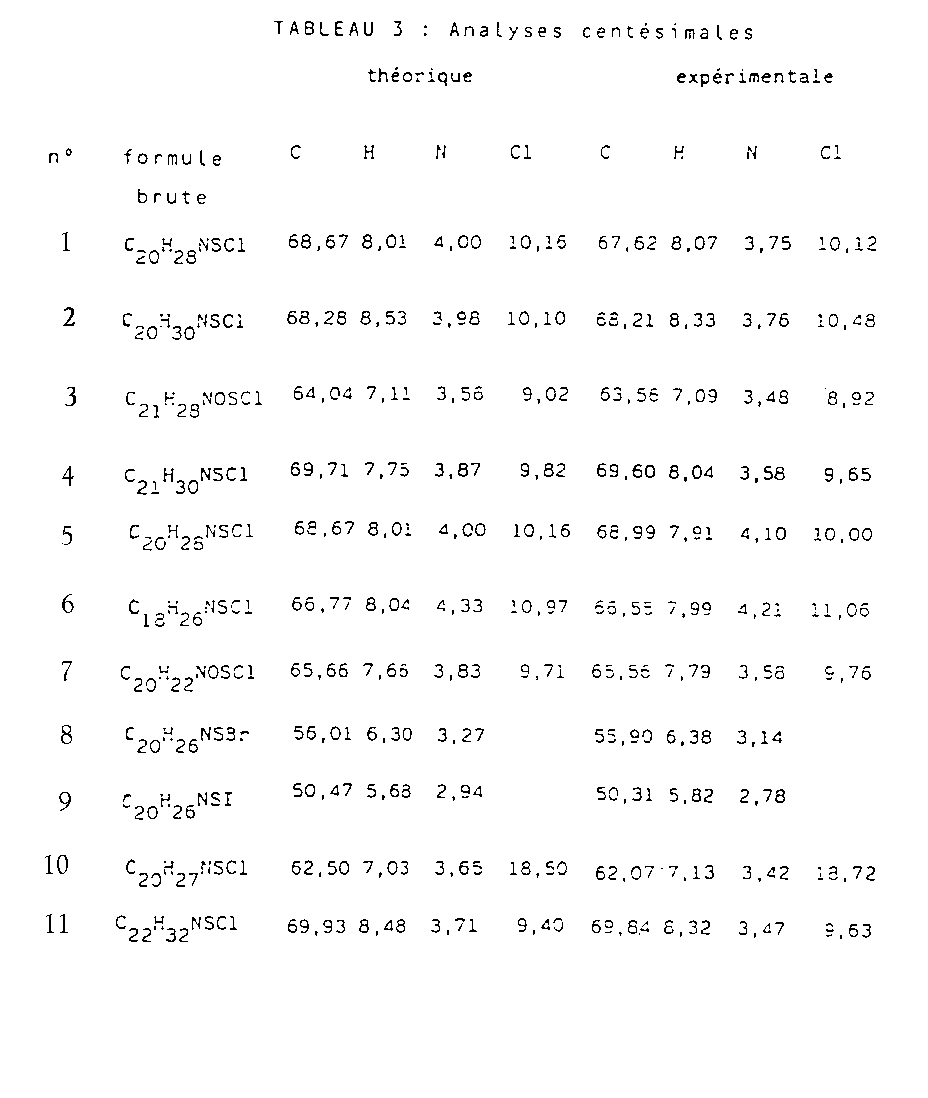

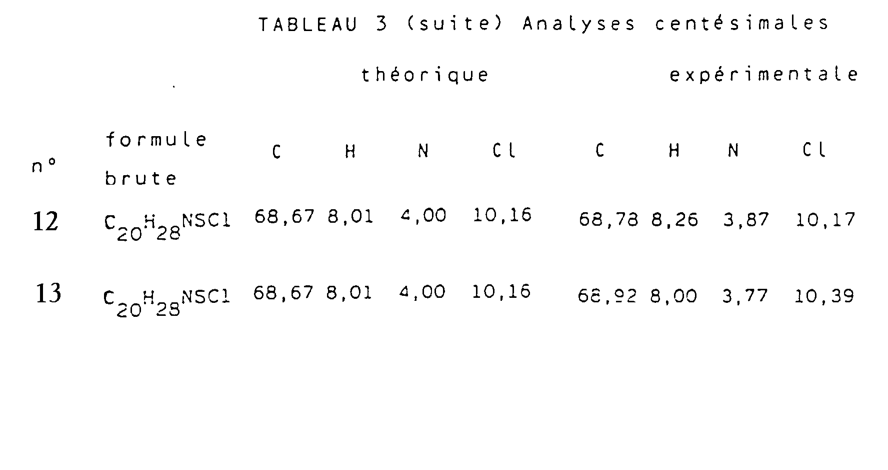

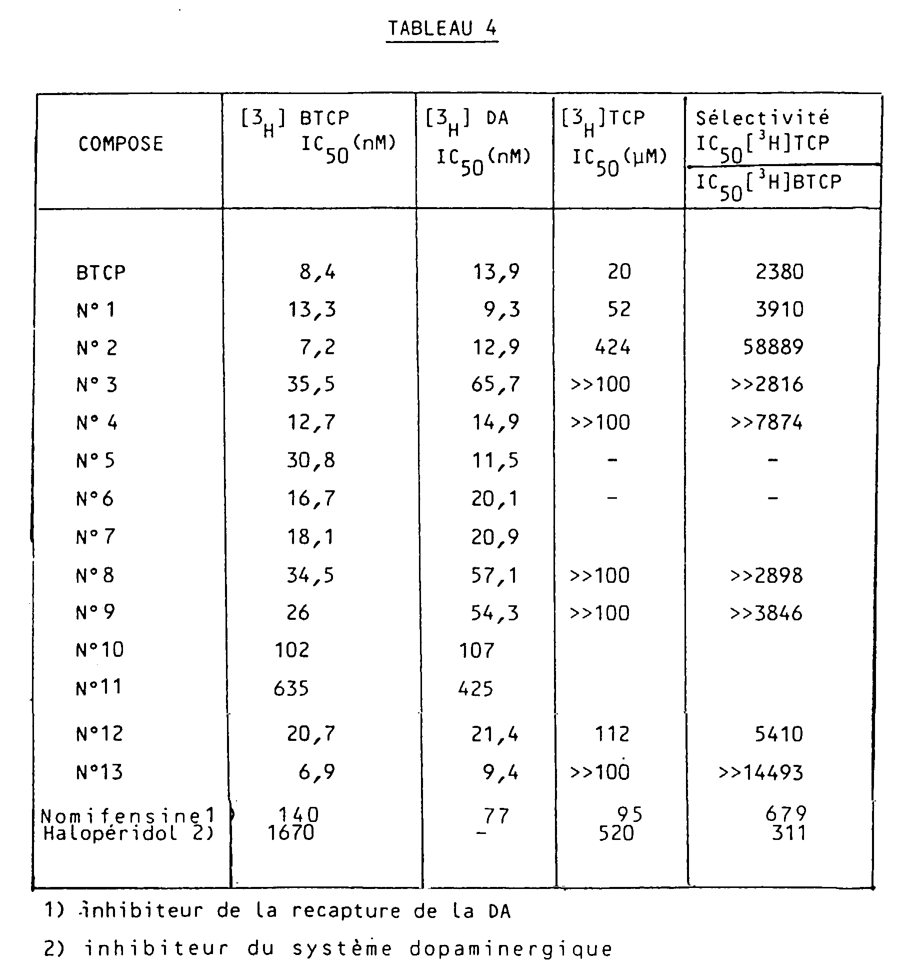

**TABLEAU 5**

| Composé | EXEMPLE 37 Capture 3H-NA IC₅₀ (nM) | Composé | EXEMPLE 38 Activité locomotrice Variation (%) par rapport aux témoins (100%) pour la dose 10 mg/kg,i.p. |
|---|---|---|---|
| BTCP | 18,3±0,7 | BTCP | +122±12 |
| N°1 | 40 ±0,5 | 1 | +160±25 |
| N°2 | 22,7±1 | 2 | +53±23 |
| N°4 | 12,5±1 | 4 | +84±12 |
| N°8 | 196±31 | 6 | +52±21 |
| N°12 | 94,3±7,3 | 7 | +416±36 |
| N°13 | 16,5±1,1 | 8 | +30±19 |
| | | 12 | +40±22 |
| | | 13 | +175±27 |

**TABLEAU 6**

| COMPOSE | Toxicité à 100 mg/kg i.p. % mortalité à 48 h |
|---|---|
| BTCP | 0 |
| N°1 | 10 |
| N°2 | 0 |
| N°4 | 10 |
| N°8 | 0 |
| N°12 | 0 |
| N°13 | 0 |

**TABLEAU 7**

| Composé | Recapture de (³H) DA | | Recapture de (³H) NA | |
|---|---|---|---|---|
| | IC 50 1) | nH 2) | IC 50 1) | nH 2) |
| | | | | |
| n°1 | 70 nM | 0,95 | - | - |
| n°2 | - | - | 6,2 µM | 0,85 |
| n°4 | 1,47 nM | 0,95 | 10,5 nM | 1,2 |
| n°13 | 8 nM | 1,07 | - | - |

| | | | | |
|---|---|---|---|---|
| 1) IC 50 est la concentration du composé qui inhibe 50% de la liaison spécifique du ligand radioactif. | | | | |
| 2) nH: nombre de Hill. | | | | |

## Revendications (Revendications pour l'(les) Etat(s) contractant(s) suivant(s): AT, BE, CH, DE, DK, FR, GB, IT, LI, LU, NL, SE)

1. Amine substituée répondant à la formule : 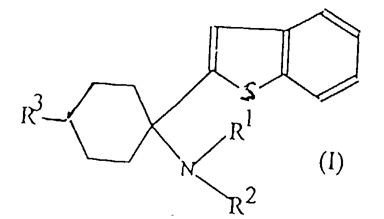 dans laquelle R¹ et R² qui peuvent être identiques ou différents, représentent un radical alkyle ou un radical alkyle substitué par au moins un substituant choisi parmi les atomes d'halogène, les radicaux alcoxy et le radical hydroxyle, ou R¹ et R² forment avec l'atome d'azote auquel ils sont liés, un cycle pipéridine comprenant éventuellement un ou plusieurs substituants choisis parmi le groupe méthyle, le groupe méthyle substitué par un atome d'halogène et le groupe 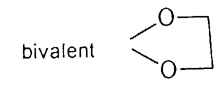 ou = 0
ou dans laquelle R¹ et R² forment avec l'atome d'azote auquel ils sont liés un cycles pipérazine de formule : 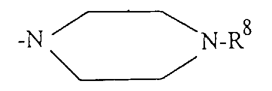 dans laquelle R⁸ représente H, un radical alkyle ou un radical alkyle substitué par au moins un substituant choisi parmi les atomes d'halogène et les groupes hydroxy, alcoxy arylalcoxy et oxycarbonylalkyle, et
- R³ est un atome d'hydrogène ou le groupe méthyle, à condition que R³ ne représente pas H lorsque R¹ et R² forment avec l'atome d'azote auquel ils sont liés un cycle pipéridine non substitué.

2. Amine substituée selon la revendication 1, caractérisée en ce que R¹ et R² forment avec l'atome d'azote auquel ils sont liés un cycle pipéridine non substitué ou substitué par au moins un groupe méthyle, un groupe méthyle substitué par un atome d'halogène et/ou le groupe 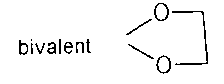 ou = 0

3. Amine substituée selon la revendication 1, caractérisée en ce que R1 et R2 sont des radicaux alkyle.

4. Amine substituée selon la revendication 3, caractérisée en ce qu'elle est le (benzo(b)thiophényl-2)-1 (dipropylamino-1)-1 cyclohexane.

5. Amine substituée selon la revendication 2, caractérisée en ce qu'elle est le (benzo(b)thiophényl-2)-1 c-méthyl-4-r-(pipéridino-1)-1 cyclohexane.

6. Amine substituée selon la revendication 2, caractérisée en ce qu'elle est le (benzo(b)thiophényl-2)-1 (méthyl-3 pipéridino)-1)-1 cyclohexane.

7. Amine substituée selon la revendication 2 choisie parmi
- le (benzo(b)thiophényl-2)-1 ((éthylènekétal-4 pipéridino)-1)-1 cyclohexane,
- le (benzo(b)thiophényl-2)-1((diméthyl-3,5 pipéridino)-1)-1 cyclohexane,
- le benzo (b) thiophényl-2) -1((bromométhyl-3 pipéridino) -1) -1 cyclohexane,
- le benzo(b)thiophényl-2)-1((iodométhyl-3 pipéridino)-1)-1 cyclohexane, et
- le (benzo(b)thiophényl-2)-1 t-méthyl-4-r-(pipéridino-1)-1 cyclohexane.

8. Procédé de préparation d'une amine substituée répondant à la formule : 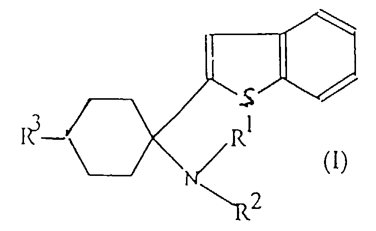 dans laquelle R¹ et R² qui peuvent être identiques ou différents, représentent un radical alkyle ou un radical alkyle substitué par au moins un substituant choisi parmi les atomes d'halogène, les radicaux alcoxy et le radical hydroxy, ou R¹ et R² forment avec l'atome d'azote auquel ils sont liés, un cycle pipéridine comprenant éventuellement un ou plusieurs substituants choisis parmi le groupe méthyle, le groupe méthyle substitué par au moins un atome d'halogène et le groupe 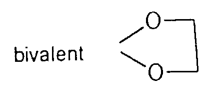 ou = 0
ou dans laquelle R¹ et R² forment avec l'atome d'azote auquel ils sont liés un cycle pipérazine de formule : 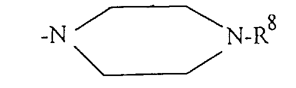 dans laquelle R⁸ représente H, un radical alkyle ou un radical alkyle substitué par au moins un substituant choisi parmi les atomes d'halogène et les groupes hydroxy, alcoxy, arylalcoxy et oxycarbonylalkyle, et
- R³ est un atome d'hydrogène ou le groupe méthyle, à condition que R³ ne représente pas H lorsque R¹ et R² forment avec l'atome d'azote auquel ils sont liés un cycle pipéridine non substitué, caractérisé en ce qu'il consiste à :
a) préparer un α-aminonitrile de formule : 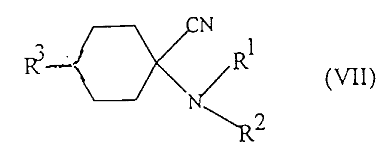 dans laquelle R¹, R² et R³ ont la signification donnée ci-dessus, et
b) faire réagir l'α-aminonitrile ainsi préparé avec un halogénure de formule:MgXR⁵ dans laquelle X représence un atome d'halogène sauf le fluor, et R⁵ représente le groupe benzothiophényle-2.

9. Procédé de préparation d'une amine substituée répondant à la formule (I) : 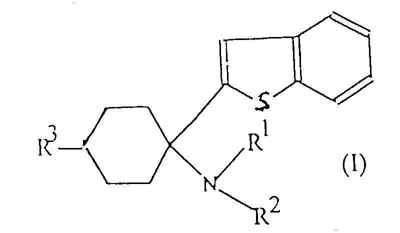 dans laquelle R¹ et R² forment avec l'atome d'azote auquel ils sont liés, un cycle pipéridine comprenant un ou plusieurs substituants constitués par le groupe méthyle substitué par un atome d'halogène, et
- R³ est un atome d'hydrogène ou le groupe méthyle, caractérisé en ce qu'il consiste à :
a) préparer un α-aminonitrile de formule : 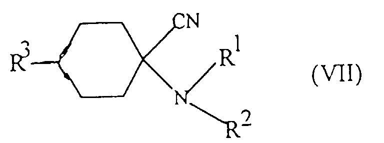 dans laquelle R³ a la signification donnée ci-dessus, et R¹ et R² forment avec l'atome d'azote auquel ils sont liés, un cycle pipéridine substitué par le radical hydroxyméthyle,
b) faire réagir l'α-aminonitrile ainsi préparé avec un halogénure de formule MgXR⁵, dans laquelle X représente un atome d'halogène sauf le fluor et R⁵ représente le groupe benzothiophényle-2, et
c) faire réagir le dérivé obtenu dans l'étape b) avec un agent halogénant ou un acide carboxylique.

10. Procédé selon la revendication 9, caractérisé en ce que l'agent halogénant est le bromure de thionyle, le chlorure de thionyle ou l'iodure de triméthylsilane.

11. Procédé de préparation d'une amine substituée de formule : 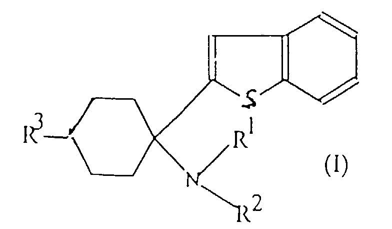 dans laquelle R¹ et R² forment avec l'atome d'azote auquel ils sont liés un cycle pipéridine comprenant éventuellement un ou plusieurs substituants choisis parmi le groupe méthyle et le groupe méthyle substitué par un atome d'halogène, et
- R³ est un atome d'hydrogène ou le groupe méthyle, à condition que R³ ne représente pas H lorsque R¹ et R² forment avec l'atome d'azote auquel ils sont liés un cycle pipéridine non substitué, caractérisé en ce qu'il consiste ;
a) à préparer un alcool de formule : 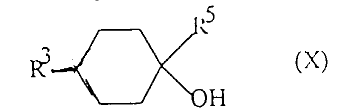 dans laquelle R³ a la signification donnée ci-dessus et R⁵ représente le groupe benzothiophényle-2,
b) à transformer l'alcool préparé dans l'étape a) en un dérivé azide correspondant de formule : 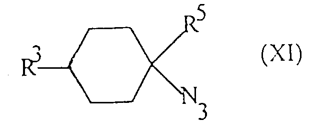
c) à réduire le dérivé azide en un mélange d'amines primaires, et
d) à faire réagir le mélange d'amines primaires avec un dihalogéno-1,5 pentane comportant éventuellement un ou plusieurs substituants choisis parmi le groupe méthyle et le groupe méthyle substitué par un atome d'halogène.

12. Composition pharmaceutique, caractérisée en ce qu'elle comprend au moins une amine substituée selon l'une quelconque des revendications 1 à 7, ou un sel d'addition à un acide de cette amine.

## Revendications (Revendications pour l'(les) Etat(s) contractant(s) suivant(s): GR)

1. Amine substituée répondant à la formule : 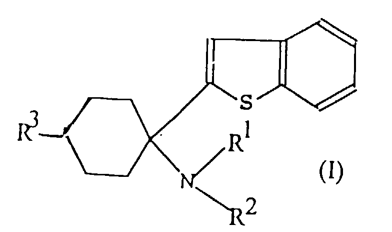 dans laquelle R¹ et R² qui peuvent être identiques ou différents, représentent un radical alkyle ou un radical alkyle substitué par au moins un substituant choisi parmi les atomes d'halogène, les radicaux alcoxy et le radical hydroxyle, ou R¹ et R² forment avec l'atome d'azote auquel ils sont liés, un cycle pipéridine comprenant éventuellement un ou plusieurs substituants choisis parmi le groupe méthyle, le groupe méthyle substitué par un atome d'halogène et le groupe 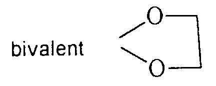 ou = 0
ou dans laquelle R¹ et R² forment avec l'atome d'azote auquel ils sont liés un cycles pipérazine de formule : 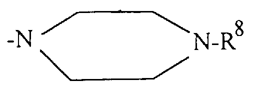 dans laquelle R⁸ représente H, un radical alkyle ou un radical alkyle substitué par au moins un substituant choisi parmi les atomes d'halogène et les groupes hydroxy, alcoxy arylalcoxy et oxycarbonylalkyle, et
- R³ est un atome d'hydrogène ou le groupe méthyle, à condition que R³ ne représente pas H lorsque R¹ et R² forment avec l'atome d'azote auquel ils sont liés un cycle pipéridine non substitué.

2. Amine substituée selon la revendication 1, caractérisée en ce que R¹ et R² forment avec l'atome d'azote auquel ils sont liés un cycle pipéridine non substitué ou substitué par au moins un groupe méthyle, un groupe méthyle substitué par un atome d'halogène et/ou le groupe 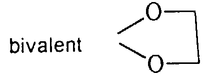 ou = 0

3. Amine substituée selon la revendication 1, caractérisée en ce que R1 et R2 sont des radicaux alkyle.

4. Amine substituée selon la revendication 3, caractérisée en ce qu'elle est le (benzo(b)thiophényl-2)-1 (dipropylamino-1)-1 cyclohexane.

5. Amine substituée selon la revendication 2, caractérisée en ce qu'elle est le (benzo(b)thiophényl-2)-1 c-méthyl-4-r-(pipéridino-1)-1 cyclohexane.

6. Amine substituée selon la revendication 2, caractérisée en ce qu'elle est le (benzo(b)thiophényl-2)-1 (méthyl-3 pipéridino)-1)-1 cyclohexane.

7. Amine substituée selon la revendication 2 choisie parmi
- le (benzo(b)thiophényl-2)-1 ((éthylènekétal-4 pipéridino)-1)-1 cyclohexane,
- le (benzo(b)thiophényl-2)-1((diméthyl-3,5 pipéridino)-1)-1 cyclohexane,
- le benzo(b)thiophényl-2)-1((bromométhyl-3 pipéridino)-1)-1 cyclohexane,
- le benzo(b) thiophényl-2) -1 ((iodométhyl-3 pipéridino)-1)-1 cyclohexane, et
- le (benzo(b)thiophényl-2)-1 t-méthyl-4-r-(pipéridino-1)-1 cyclohexane.

8. Procédé de préparation d'une amine substituée répondant à la formule : 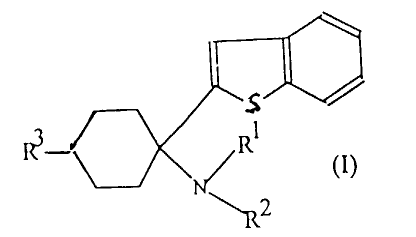 dans laquelle R¹ et R² qui peuvent être identiques ou différents, représentent un radical alkyle ou un radical alkyle substitué par au moins un substituant choisi parmi les atomes d'halogène, les radicaux alcoxy et le radical hydroxy, ou R¹ et R² forment avec l'atome d'azote auquel ils sont liés, un cycle pipéridine comprenant éventuellement un ou plusieurs substituants choisis parmi le groupe méthyle, le groupe méthyle substitué par au moins un atome d'halogène et le groupe 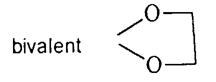 ou = 0
ou dans laquelle R¹ et R² forment avec l'atome d'azote auquel ils sont liés un cycle pipérazine de formule : 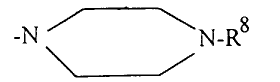 dans laquelle R⁸ représente H, un radical alkyle ou un radical alkyle substitué par au moins un substituant choisi parmi les atomes d'halogène et les groupes hydroxy, alcoxy, arylalcoxy et oxycarbonylalkyle, et
- R³ est un atome d'hydrogène ou le groupe méthyle, à condition que R³ ne représente pas H lorsque R¹ et R² forment avec l'atome d'azote auquel ils sont liés un cycle pipéridine non substitué, caractérisé en ce qu'il consiste à :
a) préparer un α-aminonitrile de formule : 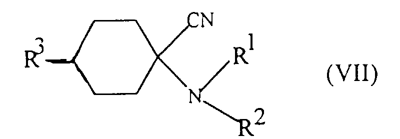 dans laquelle R¹, R² et R³ ont la signification donnée ci-dessus, et
b) faire réagir l'α-aminonitrile ainsi préparé avec un halogénure de formule:MgXR⁵ dans laquelle X représente un atome d'halogène sauf le fluor, et R⁵ représente le groupe benzothiophényle-2.

9. Procédé de préparation d'une amine substituée répondant à la formule (I): 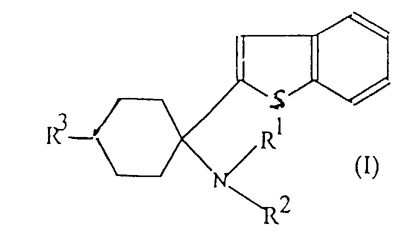 dans laquelle R¹ et R² forment avec l'atome d'azote auquel ils sont liés, un cycle pipéridine comprenant un ou plusieurs substituants constitués par le groupe méthyle substitué par un atome d'halogène, et
- R³ est un atome d'hydrogène ou le groupe méthyle, caractérisé en ce qu'il consiste à :
a) préparer un α-aminonitrile de formule : 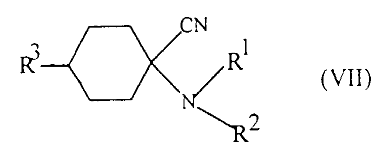 dans laquelle R³ a la signification donnée ci-dessus, et R¹ et R² forment avec l'atome d'azote auquel ils sont liés, un cycle pipéridine substitué par le radical hydroxyméthyle,
b) faire réagir l'α-aminonitrile ainsi préparé avec un halogénure de formule MgXR⁵, dans laquelle X représente un atome d'halogène sauf le fluor et R⁵ représente le groupe benzothiophényle-2, et
c) faire réagir le dérivé obtenu dans l'étape b) avec un agent halogénant ou un acide carboxylique.

10. Procédé selon la revendication 9, caractérisé en ce que l'agent halogénant est le bromure de thionyle, le chlorure de thionyle ou l'iodure de triméthylsilane.

11. Procédé de préparation d'une amine substituée de formule : 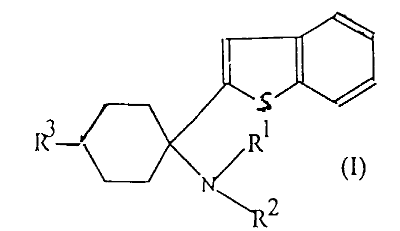 dans laquelle R¹ et R² forment avec l'atome d'azote auquel ils sont liés un cycle pipéridine comprenant éventuellement un ou plusieurs substituants choisis parmi le groupe méthyle et le groupe méthyle substitue par un atome d'halogène, et
- R³ est un atome d'hydrogène ou le groupe méthyle, à condition que R³ ne représente pas H lorsque R¹ et R² forment avec l'atome d'azote auquel ils sont liés un cycle pipéridine non substitué, caractérisé en ce qu'il consiste :
a) à préparer un alcool de formule : 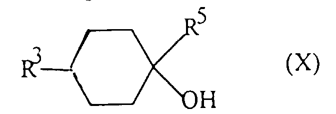 dans laquelle R³ a la signification donnée ci-dessus et R⁵ représente le groupe benzothiophényle-2,
b) à transformer l'alcool préparé dans l'étape a) en un dérivé azide correspondant de formule : 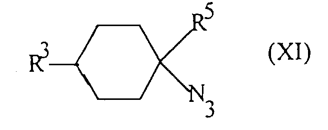
c) à réduire le dérivé azide en un mélange d'amines primaires, et
d) à faire réagir le mélange d'amines primaires avec un dihalogéno-1,5 pentane comportant éventuellement un ou plusieurs substituants choisis parmi le groupe méthyle et le groupe méthyle substitué par un atome d'halogène.

12. Procédé de préparation d'une composition pharmaceutique, caractérisée en ce qu'il consiste à mélanger au moins une amine substituée selon l'une quelconque des revendications 1 à 7, ou un sel d'addition à un acide pharmaceutiquement acceptable de cette amine, avec un diluant ou excipient pharmaceutiquement acceptable.

## Revendications (Revendications pour l'(les) Etat(s) contractant(s) suivant(s): ES)

1. Procédé de préparation d'une amine substituée répondant à la formule : 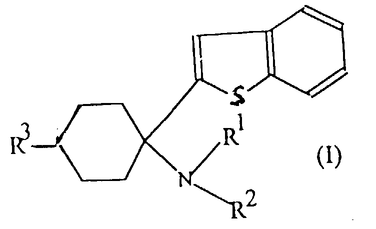 dans laquelle R¹ et R² qui peuvent être identiques ou différents, représentent un radical alkyle ou un radical alkyle substitué par au moins un substituant choisi parmi les atomes d'halogène, les radicaux alcoxy et le radical hydroxy, ou R¹ et R² forment avec l'atome d'azote auquel ils sont liés, un cycle pipéridine comprenant éventuellement un ou plusieurs substituants choisis parmi le groupe méthyle, le groupe méthyle substitué par au moins un atome d'halogène et le groupe 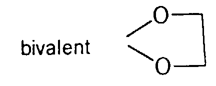 ou = 0
ou dans laquelle R¹ et R² forment avec l'atome d'azote auquel ils sont liés un cycle pipérazine de formule : 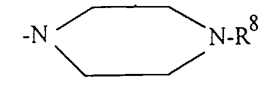 dans laquelle R⁸ représente H, un radical alkyle ou un radical alkyle substitué par au moins un substituant choisi parmi les atomes d'halogène et les groupes hydroxy, alcoxy, arylalcoxy et oxycarbonylalkyle, et
- R³ est un atome d'hydrogène ou le groupe méthyle, à condition que R³ ne représente pas H lorsque R¹ et R² forment avec l'atome d'azote auquel ils sont liés un cycle pipéridine non substitué, caractérisé en ce qu'il consiste à :
a) préparer un α-aminonitrile de formule : 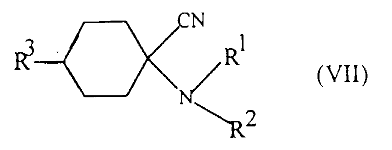 dans laquelle R¹, R² et R³ ont la signification donnée ci-dessus, et
b) faire réagir l'α-aminonitrile ainsi préparé avec un halogénure de formule:MgXR⁵ dans laquelle X représente un atome d'halogène sauf le fluor, et R⁵ représente le groupe benzothiophényle-2.

2. Procédé de préparation d'une amine substituée répondant à la formule (I) : 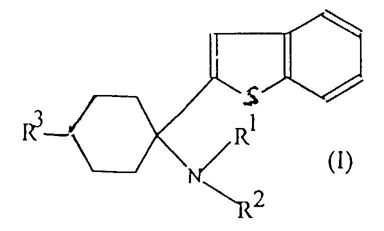 dans laquelle R¹ et R² forment avec l'atome d'azote auquel ils sont liés, un cycle pipéridine comprenant un ou plusieurs substituants constitués par le groupe méthyle substitué par un atome d'halogène, et
- R³ est un atome d'hydrogène ou le groupe méthyle, caractérisé en ce qu'il consiste à :
a) préparer un α-aminonitrile de formule : 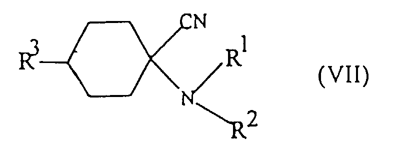 dans laquelle R³ a la signification donnée ci-dessus, et R¹ et R² forment avec l'atome d'azote auquel ils sont liés, un cycle pipéridine substitué par le radical hydroxyméthyle,
b) faire réagir l'α-aminonitrile ainsi préparé avec un halogénure de formule MgXR⁵, dans laquelle X représente un atome d'halogène sauf le fluor et R⁵ représente le groupe benzothiophényle-2, et
c) faire réagir le dérivé obtenu dans l'étape b) avec un agent halogénant ou un acide carboxylique.

3. Procédé selon la revendication 2, caractérisé en ce que l'agent halogénant est le bromure de thionyle, le chlorure de thionyle ou l'iodure de triméthylsilane.

4. Procédé de préparation d'une amine substituée de formule : 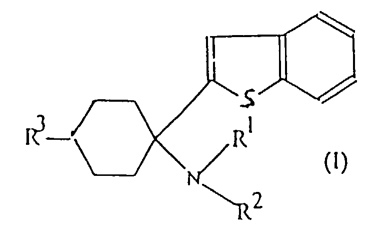 dans laquelle R¹ et R² forment avec l'atome d'azote auquel ils sont liés un cycle pipéridine comprenant éventuellement un ou plusieurs substituants choisis parmi le groupe méthyle et le groupe méthyle substitué par un atome d'halogène,
- R³ est un atome d'hydrogène ou le groupe méthyle, à condition que R³ ne représente pas H lorsque R¹ et R² forment avec l'atome d'azote auquel ils sont liés un cycle pipéridine non substitué, caractérisé en ce qu'il consiste :
a) à préparer un alcool de formule : 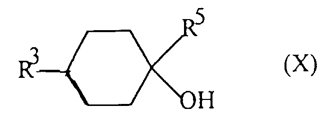 dans laquelle R³ a la signification donnée ci-dessus et R⁵ représente le groupe benzothiophényle-2,
b) à transformer l'alcool préparé dans l'étape a) en un dérivé azide correspondant de formule : 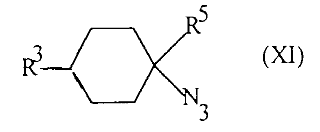
c) à réduire le dérivé azide en un mélange d'amines primaires, et
d) à faire réagir le mélange d'amines primaires avec un dihalogéno-1, 5 pentane comportant éventuellement un ou plusieurs substituants choisis parmi le groupe méthyle et le groupe méthyle substitué par un atome d'halogène.

5. Procédé selon la revendication 1, caractérisé en ce que R¹ et R² forment avec l'atome d'azote auquel ils sont liés un cycle pipéridine non substitué ou substitué par au moins un groupe méthyle, un groupe méthyle substitué par un atome d'halogène et/ou le groupe 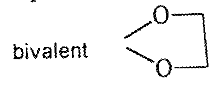 ou = 0

6. Procédé selon la revendication 1, caractérisé en ce que R¹ et R² sont des radicaux alkyle.

7. Procédé selon la revendication 6, caractérisé en ce que l'amine substituée est le (benzo(b)thiophényl-2)-1 (dipropylamino-1)-1 cyclohexane.

8. Procédé selon la revendication 2, caractérisé en ce que l'amine substituée est le (benzo(b)thiophényl-2)-1c-méthyl-4-r-(pipéiidino-1)-1 cyclohexane.

9. Procédé selon la revendication 5, caractérisé en ce que l'amine substituée est le (benzo(b)thiophényl-2)-1 (méthyl-3 pipéridino)-1)-1 cyclohexane.

10. Procédé selon la revendication 5, caractérisé en ce que l'amine substituée est choisie parmi :
- le benzo(b)thiophényl-2)-1((éthylènekétal-4 pipéridino)-1)-1 cyclohexane,
- le benzo(b)thiophényl-2)-1 ((diméthyl-3,5 pipéridino)-1)-1 cyclohexane,
- le benzo(b)thiophényl-2)-1((bromométhyl-3 pipéridino)-1)-1 cyclohexane,
- le benzo(b)thiophényl-2)-1((iodométhyl-3 pipéridino)-1)-1 cyclohexane, et
- le (benzo(b)thiophényl-2)-1 t-méthyl-4-r-(pipéridino-1)-1 cyclohexane.

11. Procédé de préparation d'une composition pharmaceutique, caractérisé en ce qu'il consiste à mélanger au moins une amine substituée répondant à la formule : 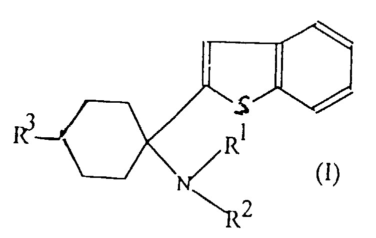 dans laquelle R¹ et R² qui peuvent être identiques ou différents, représentent un radical alkyle ou un radical alkyle substitué par au moins un substituant choisi parmi les atomes d'halogène, les radicaux alcoxy et le radical hydroxyle, ou R¹ et R² forment avec l'atome d'azote auquel ils sont liés, un cycle pipéridine comprenant éventuellement un ou plusieurs substituants choisis parmi le groupe méthyle, le groupe méthyle substitué par un atome d'halogène et le groupe 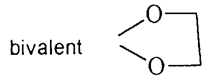 ou = 0
ou dans laquelle R¹ et R² forment avec l'atome d'azote auquel ils sont liés un cycles pipérazine de formule : 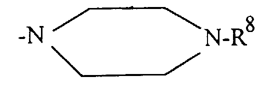 dans laquelle R⁸ représente H, un radical alkyle ou un radical alkyle substitué par au moins un substituant choisi parmi les atomes d'halogène et les groupes hydroxy, alcoxy arylalcoxy et oxycarbonylalkyle, et
- R³ est un atome d'hydrogène ou le groupe méthyle, à condition que R³ ne représente pas H lorsque R¹ et R² forment avec l'atome d'azote auquel ils sont liés un cycle pipéridine non substitué, ou un sel d'addition à un acide pharmaceutiquement acceptable de cette amine, avec un diluant ou excipient pharmaceutiquement acceptable.

12. Procédé selon la revendication 11, caractérisé en ce que l'amine substituée est choisie parmi :
- le (benzo(b)thiophény1-2)-1 ((éthylènekétal-4 pipéridino)-1)-1 cyclohexane,
- le (benzo(b)thiophényl-2)-1((diméthyl-3,5 pipéridino)-1)-1 cyclohexane,
- le benzo(b)thiophényl-2)-1((bromométhyl-3 pipéridino)-1)-1 cyclohexane,
- le benzo(b)thiophényl-2)-1((iodométhyl-3 pipéridino)-1)-1 cyclohexane, et
- le (benzo(b)thiophényl-2)-1 t-méthyl-4-r-(pipéridino-1)-1 cyclohexane.

13. Procédé selon la revendication 11, caractérisé en ce que l'amine substituée est le (benso(b)thiophényl-2)-1 (dipropylamino-1)-1 cyclohexane.

14. Procédé selon la revendication 11, caractérisé en ce que l'amine substituée est le (benzo(b)thiophényl-2)-1 c-méthyl-4-r-(pipéridino-1)-1 cyclohexane.

15. Procédé selon la revendication 11, caractérisé en ce que l'amine substituée est le (benzo(b)thiophényl-2)-1 (méthyl-3 pipéridino)-1)-1 cyclohexane.

## Patentansprüche (Patentansprüche für folgende(n) Vertragsstaat(en): AT, BE, CH, DE, DK, FR, GB, IT, LI, LU, NL, SE)

1. Substituiertes Amin mit der Formel: 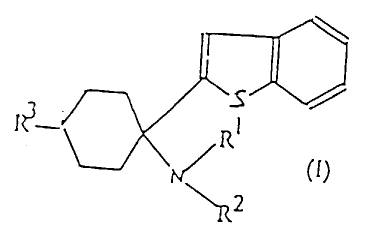 worin R¹ und R², welche gleich oder verschieden sein können, einen Alkylrest oder einen Alkylrest, der durch mindestens einen Substituenten, gewählt aus den Halogenatomen, den Alkoxyresten und dem Hydroxylrest, substituiert ist, darstellen, oder R¹ und R² mit dem Stickstoffatom, an das sie gebunden sind, einen Piperidinring bilden, welcher gegebenenfalls einen oder mehrere Substituenten, gewählt aus der Methylgruppe, der Methylgruppe, die durch ein Halogenatom substituiert ist, und der zweiwertigen Gruppe 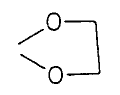 oder = 0,
umfaßt, oder worin R¹ und R² mit dem Stickstoffatom, an das sie gebunden sind, einen Piperazinring der Formel: 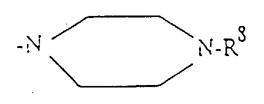 bilden, worin R⁸ H, einen Alkylrest oder einen Alkylrest, der durch mindestens einen Substituenten, gewählt aus den Halogenatomen und den Hydroxygruppen, Alkoxygruppen, Arylalkoxygruppen und Oxycarbonylalkylgruppen, substituiert ist, darstellt, und
R³ ein Wasserstoffatom oder eine Methylgruppe ist, mit dem Vorbehalt, daß R³ nicht H darstellt, wenn R¹ und R² mit dem Stickstoffatom, an das sie gebunden sind, einen nichtsubstituierten Piperidinring bilden.

2. Substituiertes Amin nach Anspruch 1, **dadurch gekennzeichnet**, daß R¹ und R² mit dem Stickstoffatom, an das sie gebunden sind, einen nichtsubstituierten oder durch mindestens eine Methylgruppe, eine Methylgruppe, die durch ein Halogenatom substituiert ist und/oder die zweiwertige Gruppe 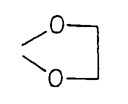 oder = 0
substituierten Piperidinring bilden.

3. Substituiertes Amin nach Anspruch 1, **dadurch gekennzeichnet,** daß R¹ und R² Alkylreste sind.

4. Substituiertes Amin nach Anspruch 3, **dadurch gekennzeichnet,** daß es das 1-(Benzo(b)thiophen-2-yl)-1-(1-dipropylamino)-cyclohexan ist.

5. Substituiertes Amin nach Anspruch 2, **dadurch gekennzeichnet,** daß es das 1-(Benzo(b)thiophen-2-yl)-4-c-methyl-1r-(1-piperidino)-cyclohexan ist.

6. Substituiertes Amin nach Anspruch 2, **dadurch gekennzeichnet**, daß es das 1-(Benzo(b)thiophen-2-yl)-1-(1-(3-methylpiperidino))-cyclohexan ist.

7. Substituiertes Amin nach Anspruch 2, gewählt aus
(1-(Benzo(b)thiophen-2-yl)-1-(1-(4-ethylenketalpiperidino))-cyclohexan,
1-(Benzo(b)thiophen-2-yl)-1-(1-(3,5-dimethylpiperidino))-cyclohexan,
1-(Benzo(b)thiophen-2-yl)-1-(1-(3-brommethylpiperidino))-cyclohexan,
1-(Benzo(b)thiophen-2-yl)-1-(1-(3-iodmethylpiperidino))-cyclohexan, und
1-(Benzo(b)thiophen-2-yl)-4-t-methyl-1r-(1-piperidino)-cyclohexan.

8. Verfahren zum Herstellen eines substituierten Amins mit der Formel: 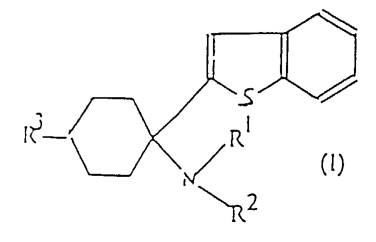 worin R¹ und R², welche gleich oder verschieden sein können, einen Alkylrest oder einen Alkylrest, der durch mindestens einen Substituenten, gewählt aus den Halogenatomen, den Alkoxyresten und dem Hydroxyrest substituiert ist, darstellen, oder R¹ und R² mit dem Stickstoffatom, an das sie gebunden sind, einen Piperidinring bilden, welcher gegebenenfalls einen oder mehrere Substituenten, gewählt aus der Methylgruppe, der Methylgruppe, die durch mindestens ein Halogenatom substituiert ist, und der zweiwertigen Gruppe 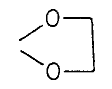 oder = 0
umfaßt,
oder worin R¹ und R² mit dem Stickstoffatom, an das sie gebunden sind, einen Piperazinring der Formel: 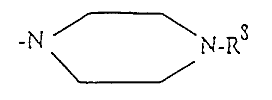 bilden, worin R⁸ H, einen Alkylrest oder einen Alkylrest, der mit mindestens einem Substituenten, gewählt aus den Halogenatomen und den Hydroxygruppen, Alkoxygruppen, Arylalkoxygruppen und Oxycarbonylalkylgruppen, substituiert ist, darstellt, und
R³ ein Wasserstoffatom oder eine Methylgruppe ist,
mit dem Vorbehalt, daß R³ nicht H darstellt, wenn R¹ und R² mit dem Stickstoffatom, an das sie gebunden sind, einen nichtsubstituierten Piperidinring bilden, **dadurch gekennzeichnet**, daß es umfaßt:
a) Herstellen eines α-Aminonitrils der Formel: 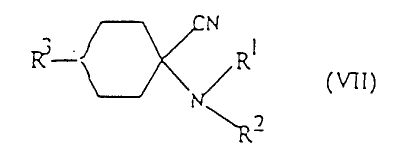 worin R¹, R² und R³ die vorstehend angegebene Bedeutung haben, und
b) Umsetzen des so hergestellten α-Aminonitrils mit einem Halogenid der Formel MgXR⁵, worin X ein Halogenatom mit Ausnahme von Fluor darstellt, und R⁵ die Gruppe Benzothiophen-2-yl darstellt.

9. Verfahren zum Herstellen eines substituierten Amins mit der Formel (I): 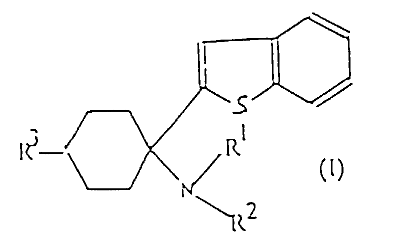
worin R¹ und R² mit dem Stickstoffatom, an das sie gebunden sind, einen Piperidinring bilden, der einen oder mehrere Substituenten umfaßt, die aus einer mit einem Halogenatom substituierten Methylgruppe bestehen, und
R³ ein Wasserstoffatom oder eine Methylgruppe ist,
**dadurch gekennzeichnet**, daß es umfaßt:
a) Herstellen eines α-Aminonitrils mit der Formel: 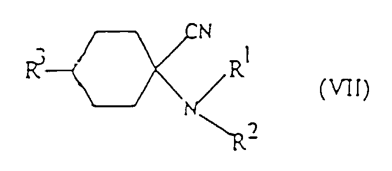
worin R³ die vorstehend angegebene Bedeutung hat,
und R¹ und R² mit dem Stickstoffatom, an das sie gebunden sind, einen Piperidinring bilden, der durch einen Hydroxymethylrest substituiert ist,
b) Umsetzen des so erhaltenen α-Aminonitrils mit einem Halogenid der Formel MgXR⁵, worin X ein Halogenatom mit Ausnahme von Fluor darstellt, und R⁵ die Benzothiophen-2-yl-Gruppe darstellt, und
c) Umsetzen des in Schritt b) erhaltenen Derivats mit einem Halogenierungsmittel oder einer Carbonsäure.

10. Verfahren nach Anspruch 9, **dadurch gekennzeichnet**, daß das Halogenierungsmittel Thionylbromid, Thionylchlorid oder Iodtrimethylsilan ist.

11. Verfahren zum Herstellen eines substituierten Amins der Formel: 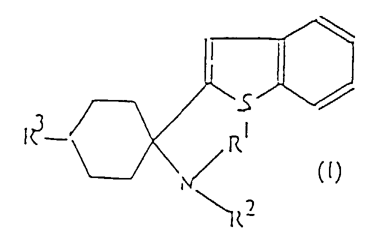
worin R¹ und R² mit dem Stickstoffatom, an das sie gebunden sind, einen Piperidinring bilden, welcher gegebenenfalls einen oder mehrere Substituenten, gewählt aus der Methylgruppe und der durch ein Halogenatom substituierten Methylgruppe, umfaßt, und
R³ ein Wasserstoffatom oder eine Methylgruppe ist,
mit dem Vorbehalt, daß R³ nicht H darstellt, wenn R¹ und R² mit dem Stickstoffatom, an das sie gebunden sind, einen nichtsubstituierten Piperidinring bilden,
**dadurch gekennzeichnet**, daß es umfaßt:
a) Herstellen eines Alkohols der Formel: 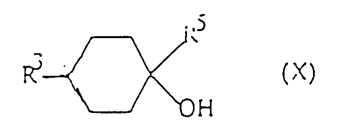 worin R³ die vorstehend angegebene Bedeutung hat und R⁵ die Benzothiophen-2-yl-Gruppe darstellt,
b) Umwandeln des in Schritt a) hergestellten Alkohols in ein Azidderivat mit der Formel: 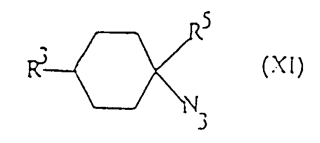
c) Reduzieren des Azidderivats zu einem Gemisch aus primären Aminen, und
d) Umsetzen des Gemischs aus primären Aminen mit einem 1,5-Dihalogen-pentan, welches gegebenenfalls einen oder mehrere Substituenten, gewählt aus der Methylgruppe und der durch ein Halogenatom substituierten Methylgruppe, trägt.

12. Pharmazeutische Zusammensetzung, **dadurch gekennzeichnet**, daß sie mindestens ein substituiertes Amin nach einem der Ansprüche 1 bis 7 oder ein Säureadditionssalz dieses Amins umfaßt.

## Patentansprüche (Patentansprüche für folgende(n) Vertragsstaat(en): GR)

1. Substituiertes Amin mit der Formel: 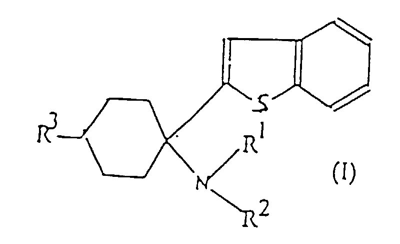 worin R¹ und R², welche gleich oder verschieden sein können, einen Alkylrest oder einen Alkylrest, der durch mindestens einen Substituenten, gewählt aus den Halogenatomen, den Alkoxyresten und dem Hydroxylrest, substituiert ist, darstellen, oder R¹ und R² mit dem Stickstoffatom, an das sie gebunden sind, einen Piperidinring bilden, welcher gegebenenfalls einen oder mehrere Substituenten, gewählt aus der Methylgruppe, der Methylgruppe, die durch ein Halogenatom substituiert ist, und der zweiwertigen Gruppe 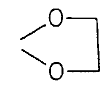 oder = 0,
umfaßt, oder worin R¹ und R² mit dem Stickstoffatom, an das sie gebunden sind, einen Piperazinring der Formel: 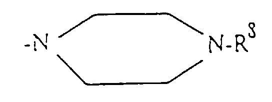 bilden, worin R⁸ H, einen Alkylrest oder einen Alkylrest, der durch mindestens einen Substituenten, gewählt aus den Halogenatomen und den Hydroxygruppen, Alkoxygruppen, Arylalkoxygruppen und Oxycarbonylalkylgruppen, substituiert ist, darstellt, und
R³ ein Wasserstoffatom oder eine Methylgruppe ist, mit dem Vorbehalt, daß R³ nicht H darstellt, wenn R¹ und R² mit dem Stickstoffatom, an das sie gebunden sind, einen nichtsubstituierten Piperidinring bilden.

2. Substituiertes Amin nach Anspruch 1, **dadurch gekennzeichnet**, daß R¹ und R² mit dem Stickstoffatom, an das sie gebunden sind, einen nichtsubstituierten oder durch mindestens eine Methylgruppe, eine Methylgruppe, die durch ein Halogenatom substituiert ist, und/oder die zweiwertige Gruppe 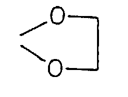 oder = 0
substituierten Piperidinring bilden.

3. Substituiertes Amin nach Anspruch 1, **dadurch gekennzeichnet**, daß R¹ und R² Alkylreste sind.

4. Substituiertes Amin nach Anspruch 3, **dadurch gekennzeichnet**, daß es das 1-(Benzo(b)thiophen-2-yl)-1-(1-dipropylamino)-cyclohexan ist.

5. Substituiertes Amin nach Anspruch 2, **dadurch gekennzeichnet**, daß es das 1-(Benzo(b)thiophen-2-yl)-4-c-methyl-1r-(1-piperidino)-cyclohexan ist.

6. Substituiertes Amin nach Anspruch 2, **dadurch gekennzeichnet**, daß es das l-(Benzo(b)thiophen-2-yl)-1-(1-(3-methylpiperidino))-cyclohexan ist.

7. Substituiertes Amin nach Anspruch 2, gewählt aus
(1-(Benzo(b)thiophen-2-yl)-1-(1-(4-ethylenketalpiperidino))-cyclohexan,
1-(Benzo(b)thiophen-2-yl)-1-(1-(3,5-dimethylpiperidino))-cyclohexan,
1-(Benzo(b)thiophen-2-yl)-l-(1-(3-brommethylpiperidino))-cyclohexan,
1-(Benzo(b)thiophen-2-yl)-1-(1-(3-iodmethylpiperidino))-cyclohexan, und
1-(Benzo(b)thiophen-2-yl)-4-t-methyl-1r-(1-piperidino)-cyclohexan.

8. Verfahren zum Herstellen eines substituierten Amins mit der Formel: 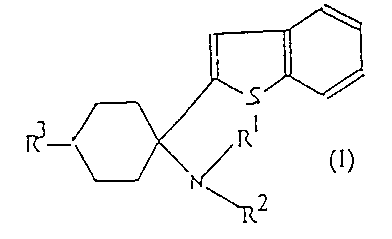 worin R¹ und R², welche gleich oder verschieden sein können, einen Alkylrest oder einen Alkylrest, der durch mindestens einen Substituenten, gewählt aus den Halogenatomen, den Alkoxyresten und dem Hydroxyrest substituiert ist, darstellen, oder R¹ und R² mit dem Stickstoffatom, an das sie gebunden sind, einen Piperidinring bilden, welcher gegebenenfalls einen oder mehrere Substituenten, gewählt aus der Methylgruppe, der Methylgruppe, die durch mindestens ein Halogenatom substituiert ist, und der zweiwertigen Gruppe 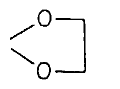 oder = 0
umfaßt,
oder worin R¹ und R² mit dem Stickstoffatom, an das sie gebunden sind, einen Piperazinring der Formel: 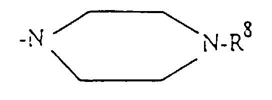 bilden, worin R⁸ H, einen Alkylrest oder einen Alkylrest, der mit mindestens einem Substituenten gewählt aus den Halogenatomen und den Hydroxygruppen, Alkoxygruppen, Arylalkoxygruppen und Oxycarbonylalkylgruppen, substituiert ist, darstellt, und
R³ ein Wasserstoffatom oder eine Methylgruppe ist,
mit dem Vorbehalt, daß R³ nicht H darstellt, wenn R¹ und R² mit dem Stickstoffatom, an das sie gebunden sind, einen nichtsubstituierten Piperidinring bilden, **dadurch gekennzeichnet**, daß es umfaßt:
a) Herstellen eines α-Aminonitrils der Formel: 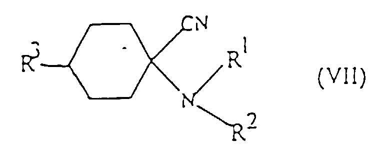 worin R¹, R² und R³ die vorstehend angegebene Bedeutung haben, und
b) Umsetzen des so hergestellten α-Aminonitrils mit einem Halogenid der Formel MgXR⁵, worin X ein Halogenatom mit Ausnahme von Fluor darstellt, und R⁵ die Gruppe Benzothiophen-2-yl darstellt.

9. Verfahren zum Herstellen eines substituierten Amins mit der Formel (I) : 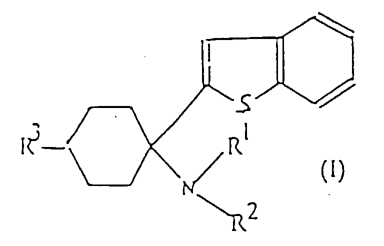
worin R¹ und R² mit dem Stickstoffatom, an das sie gebunden sind, einen Piperidinring bilden, der einen oder mehrere Substituenten umfaßt, die aus einer mit einem Halogenatom substituierten Methylgruppe bestehen, und
R³ ein Wasserstoffatom oder eine Methylgruppe ist,
**dadurch gekennzeichnet**, daß es umfaßt:
a) Herstellen eines α-Aminonitrils mit der Formel: 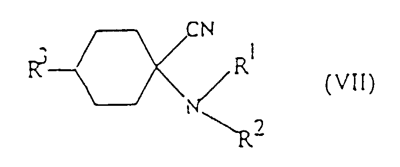 worin R³ die vorstehend angegebene Bedeutung hat, und R¹ und R² mit dem Stickstoffatom, an das sie gebunden sind, einen Piperidinring bilden, der durch einen Hydroxymethylrest substituiert ist,
b) Umsetzen des so erhaltenen α-Aminonitrils mit einem Halogenid der Formel MgXR⁵, worin X ein Halogenatom mit Ausnahme von Fluor darstellt, und R⁵ die Benzothiophen-2-yl-Gruppe darstellt, und
c) Umsetzen des in Schritt b) erhaltenen Derivats mit einem Halogenierungsmittel oder einer Carbonsäure.

10. Verfahren nach Anspruch 9, **dadurch gekennzeichnet**, daß das Halogenierungsmittel Thionylbromid, Thionylchlorid oder Iodtrimethylsilan ist.

11. Verfahren zum Herstellen eines substituierten Amins der Formel: 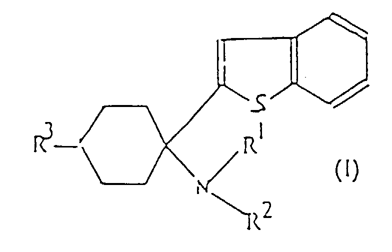
worin R¹ und R² mit dem Stickstoffatom, an das sie gebunden sind, einen Piperidinring bilden, welcher gegebenenfalls einen oder mehrere Substituenten, gewählt aus der Methylgruppe und der durch ein Halogenatom substituierten Methylgruppe, umfaßt, und
R³ ein Wasserstoffatom oder eine Methylgruppe ist,
mit dem Vorbehalt, daß R³ nicht H darstellt, wenn R¹ und R² mit dem Stickstoffatom, an das sie gebunden sind, einen nichtsubstituierten Piperidinring bilden,
**dadurch gekennzeichnet**, daß es umfaßt:
a) Herstellen eines Alkohols der Formel: 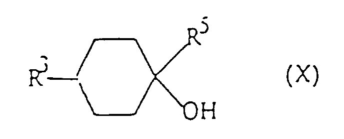 worin R³ die vorstehend angegebene Bedeutung hat und R⁵ die Benzothiophen-2-yl-Gruppe darstellt,
b) Umwandeln des in Schritt a) hergestellten Alkohols in ein Azidderivat mit der Formel: 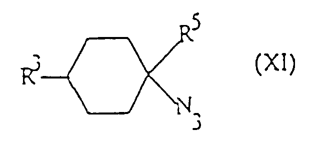
c) Reduzieren des Azidderivats zu einem Gemisch aus primären Aminen, und
d) Umsetzen des Gemischs aus primären Aminen mit einem 1,5-Dihalogen-pentan, welches gegebenenfalls einen oder mehrere Substituenten, gewählt aus der Methylgruppe und der durch ein Halogenatom substituierten Methylgruppe, trägt.

12. Verfahren zum Herstellen einer pharmazeutischen Zusammensetzung, **dadurch gekennzeichnet**, daß es das Vermischen von mindestens einem substituierten Amin nach einem der Ansprüche 1 bis 7 oder einem pharmazeutisch annehmbaren Säureadditionssalz dieses Amins mit einem pharmazeutisch annehmbaren verdünnungsmittel oder Excipiens umfaßt.

## Patentansprüche (Patentansprüche für folgende(n) Vertragsstaat(en): ES)

1. Verfahren zum Herstellen eines substituierten Amins mit der Formel: 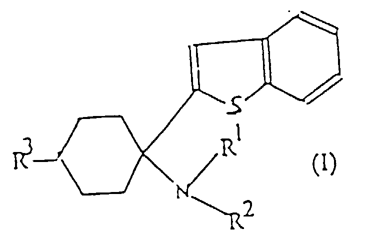 worin R¹ und R², welche gleich oder verschieden sein können, einen Alkylrest oder einen Alkylrest, der durch mindestens einen Substituenten, gewählt aus den Halogenatomen, den Alkoxyresten und dem Hydroxylrest, substituiert ist, darstellen, oder R¹ und R² mit dem Stickstoffatom, an das sie gebunden sind, einen Piperidinring bilden, welcher gegebenenfalls einen oder mehrere Substituenten, gewählt aus der Methylgruppe, der Methylgruppe, die durch mindestens ein Halogenatom substituiert ist, und der zweiwertigen Gruppe 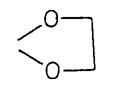 oder = 0,
umfaßt, oder worin R¹ und R² mit dem Stickstoffatom, an das sie gebunden sind, einen Piperazinring der Formel: 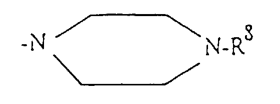 bilden, worin R⁸ H, einen Alkylrest oder einen Alkylrest, der durch mindestens einen Substituenten gewählt aus den Halogenatomen und den Hydroxygruppen, Alkoxygruppen, Arylalkoxygruppen und Oxycarbonylalkylgruppen, substituiert ist, darstellt, und
R³ ein Wasserstoffatom oder eine Methylgruppe ist, mit dem Vorbehalt, daß R³ nicht H darstellt, wenn R¹ und R² mit dem Stickstoffatom, an das sie gebunden sind, einen nichtsubstituierten Piperidinring bilden, **dadurch gekennzeichnet**, daß es umfaßt:
a) Herstellen eines α-Aminonitrils der Formel: 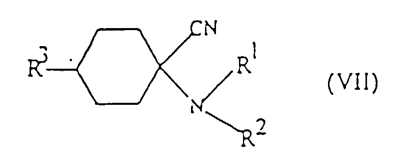 worin R¹, R² und R³ die vorstehend angegebene Bedeutung haben, und
b) Umsetzen des so hergestellten α-Aminonitrils mit einem Halogenid der Formel MgXR⁵, worin X ein Halogenatom mit Ausnahme von Fluor darstellt, und R⁵ die Gruppe Benzothiophen-2-yl darstellt.

2. Verfahren zum Herstellen eines substituierten Amins mit der Formel (I) : 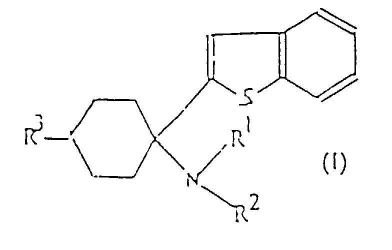
worin R¹ und R² mit dem Stickstoffatom, an das sie gebunden sind, einen Piperidinring bilden, der einen oder mehrere Substituenten umfaßt, die aus einer mit einem Halogenatom substituierten Methylgruppe bestehen, und
R³ ein Wasserstoffatom oder eine Methylgruppe ist,
**dadurch gekennzeichnet**, daß es umfaßt:
a) Herstellen eines α-Aminonitrils mit der Formel: 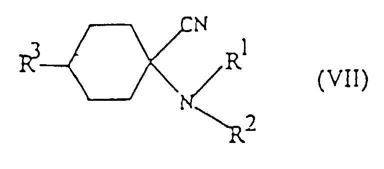 worin R³ die vorstehend angegebene Bedeutung hat, und R¹ und R² mit dem Stickstoffatom, an das sie gebunden sind, einen Piperidinring bilden, der durch einen Hydroxymethylrest substituiert ist,
b) Umsetzen des so erhaltenen α-Aminonitrils mit einem Halogenid der Formel MgXR⁵, worin X ein Halogenatom mit Ausnahme von Fluor darstellt, und R⁵ die Benzothiophen-2-yl-Gruppe darstellt, und
c) Umsetzen des in Schritt b) erhaltenen Derivats mit einem Halogenierungsmittel oder einer Carbonsäure.

3. Verfahren nach Anspruch 2, **dadurch gekennzeichnet**, daß das Halogenierungsmittel Thionylbromid, Thionylchlorid oder Iodtrimethylsilan ist.

4. Verfahren zum Herstellen eines substituierten Amins der Formel: 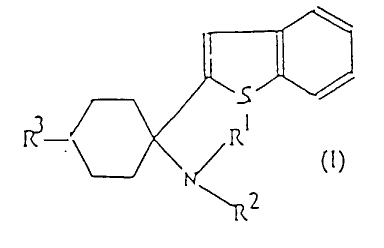
worin R¹ und R² mit dem Stickstoffatom, an das sie gebunden sind, einen Piperidinring bilden, welcher gegebenenfalls einen oder mehrere Substituenten, gewählt aus der Methylgruppe und der durch ein Halogenatom substituierten Methylgruppe, umfaßt, und
R³ ein Wasserstoffatom oder eine Methylgruppe ist,
mit dem Vorbehalt, daß R³ nicht H darstellt, wenn R¹ und R² mit dem Stickstoffatom, an das sie gebunden sind, einen nichtsubstituierten Piperidinring bilden,
**dadurch gekennzeichnet**, daß es umfaßt:
a) Herstellen eines Alkohols der Formel: 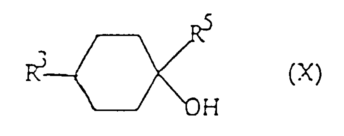 worin R³ die vorstehend angegebene Bedeutung hat und R⁵ die Benzothiophen-2-yl-Gruppe darstellt,
b) Umwandeln des in Schritt a) hergestellten Alkohols in ein Azidderivat mit der Formel: 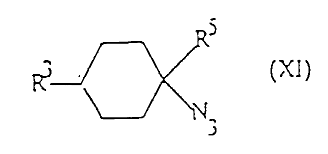
c) Reduzieren des Azidderivats zu einem Gemisch aus primären Aminen, und
d) Umsetzen des Gemischs aus primären Aminen mit einem 1,5-Dihalogen-pentan, welches gegebenenfalls einen oder mehrere Substituenten, gewählt aus der Methylgruppe und der durch ein Halogenatom substituierten Methylgruppe, trägt.

5. Verfahren nach Anspruch 1, **dadurch gekennzeichnet**, daß R¹ und R² mit dem Stickstoffatom, an das sie gebunden sind, einen nichtsubstituierten oder durch mindestens eine Methylgruppe, eine Methylgruppe, die durch ein Halogenatom substituiert ist, und/oder die zweiwertige Gruppe 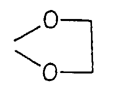 oder = 0
substituierten Piperidinring bilden.

6. Verfahren nach Anspruch 1, **dadurch gekennzeichnet**, daß R¹ und R² Alkylreste sind.

7. Verfahren nach Anspruch 6, **dadurch gekennzeichnet**, daß das substituierte Amin das 1-(Benzo(b)thiophen-2-yl)-1-(1-dipropylamino)-cyclohexan ist.

8. Verfahren nach Anspruch 2, **dadurch gekennzeichnet**, daß das substituierte Amin das 1-(Benzo(b)thiophen-2-yl)-4-c-methyl-1r-(1-piperidino)-cyclohexan ist.

9. Verfahren nach Anspruch 5, **dadurch gekennzeichnet**, daß das substituierte Amin das 1-(Benzo(b)thiophen-2-yl)-1-(1-(3-methylpiperidino))-cyclohexan ist.

10. Verfahren nach Anspruch 5, **dadurch gekennzeichnet**, daß das substituierte Amin gewählt ist aus:
(1-(Benzo(b)thiophen-2-yl)-1-(1-(4-ethylenketalpiperidino))-cyclohexan,
1-(Benzo(b)thiophen-2-yl)-1-(1-(3,5-dimethylpiperidino))-cyclohexan,
1-(Benzo(b)thiophen-2-yl)-1-(1-(3-brommethylpiperidino))-cyclohexan,
1-(Benzo(b)thiophen-2-yl)-1-(1-(3-iodmethylpiperidino))-cyclohexan, und
1-(Benzo(b)thiophen-2-yl)-4-t-methyl-1r-(1-piperidino)-cyclohexan.

11. Verfahren zum Herstellen einer pharmazeutischen Zusammensetzung, **dadurch gekennzeichnet**, daß es das Vermischen von mindestens einem substituierten Amin mit der Formel: 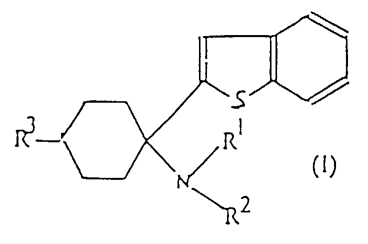 worin R¹ und R², welche gleich oder verschieden sein können, einen Alkylrest oder einen Alkylrest, der durch mindestens einen Substituenten, gewählt aus den Halogenatomen, den Alkoxyresten und dem Hydroxylrest, substituiert ist, darstellen, oder R¹ und R² mit dem Stickstoffatom, an das sie gebunden sind, einen Piperidinring bilden, welcher gegebenenfalls einen oder mehrere Substituenten, gewählt aus der Methylgruppe, der Methylgruppe, die durch ein Halogenatom substituiert ist, und der zweiwertigen Gruppe 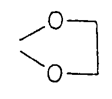 oder = 0
umfaßt, oder worin R¹ und R² mit dem Stickstoffatom, an das sie gebunden sind, einen Piperazinring der Formel: 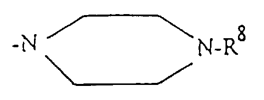 bilden, worin R⁸ H, einen Alkylrest oder einen Alkylrest, der durch mindestens einen Substituenten, gewählt aus den Halogenatomen und den Hydroxygruppen, Alkoxygruppen, Arylalkoxygruppen und Oxycarbonylalkylgruppen, substituiert ist, darstellt, und
R³ ein Wasserstoffatom oder eine Methylgruppe ist, mit dem Vorbehalt, daß R³ nicht H darstellt, wenn R¹ und R² mit dem Stickstoffatom, an das sie gebunden sind, einen nichtsubstituierten Piperidinring bilden,
oder von einem pharmazeutisch annehmbaren Säureadditionssalz dieses Amins mit einem pharmazeutisch annehmbaren Verdünnungsmittel oder Excipienz umfaßt.

12. Verfahren nach Anspruch 11, **dadurch gekennzeichnet**, daß das substituierte Amin gewählt ist aus:
(1-(Benzo(b)thiophen-2-yl)-1-(1-(4-ethylenketalpiperidino))-cyclohexan,
1-(Benzo(b)thiophen-2-yl)-1-(1-(3,5-dimethylpiperidino))-cyclohexan,
1-(Benzo(b)thiophen-2-yl)-1-(1-(3-brommethylpiperidino))-cyclohexan,
1-(Benzo(b)thiophen-2-yl)-1-(1-(3-iodmethylpiperidino))-cyclohexan, und
1-(Benzo(b)thiophen-2-yl)-4-t-methyl-1r-(1-piperidino)-cyclohexan.

13. Verfahren nach Anspruch 11, **dadurch gekennzeichnet**, daß das substituierte Amin das 1-(Benzo(b)thiophen-2-yl)-1-(1-dipropylamino)-cyclohexan ist.

14. Verfahren nach Anspruch 11, **dadurch gekennzeichnet**, daß das substituierte Amin das 1-(Benzo(b)thiophen-2-yl)-4-c-methyl-1r-(1-piperidino)-cyclohexan ist.

15. Verfahren nach Anspruch 11, **dadurch gekennzeichnet**, daß das substituierte Amin das 1-(Benzo(b)thiophen-2-yl)-1-(1-(3-methylpiperidino))-cyclohexan ist.

## Claims (Claims for the following Contracting State(s): AT, BE, CH, DE, DK, FR, GB, IT, LI, LU, NL, SE)

1. Substituted amine complying with the formula: 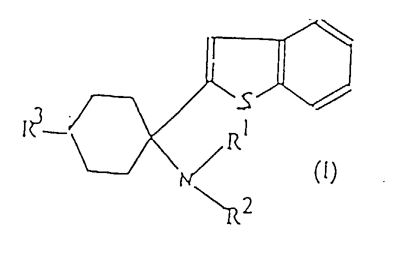 in which R¹ and R², which can be the same or different, represent an alkyl radical or an alkyl radical substituted by at least one substituent chosen from among halogen atoms, alkoxy radicals and the hydroxyl radical, where R¹ and R² form with the nitrogen atom to which they are bonded a piperidine cycle optionally having one or more substituents chosen from among the methyl group, the methyl group substituted by a halogen atom and the group 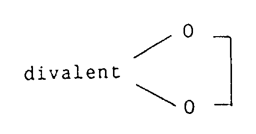 or = 0
in which R¹ and R² form with the nitrogen atom to which they are bonded a piperazine cycle of formula: 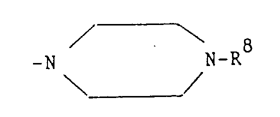 in which R⁸ represents H, an alkyl radical or an alkyl radical substituted by at least one substituent chosen from among halogen atoms and hydroxy, alkoxy, arylalkoxy and oxycarbonylalkyl groups and R³ is a hydrogen atom or the methyl group, provided that R³ does not represent H when R¹ and R² form with the nitrogen atom to which they are bonded an unsubstituted piperidine cycle.

2. Substituted amine according to claim 1, characterized in that R¹ and R² form with the nitrogen atom to which they are bonded an unsubstituted piperidine cycle or a piperidine cycle substituted by at least one methyl group, a methyl group substituted by a halogen atom and/or the group 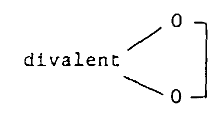 or = 0

3. Substituted amine according to claim 1, characterized in that R¹ and R² are alkyl radicals.

4. Substituted amine according to claim 3, characterized in that it is the (benzo(b)thiophenyl-2)-1 (dipropylamino-1)-1-cyclohexane.

5. Substituted amine according to claim 2, characterized in that it is the (benzo(b)thiophenyl-2)-1 c-methyl-4-r-(piperidino-1)-1-cyclohexane.

6. Substituted amine according to claim 2, characterized in that it is the (benzo(b)thiophenyl-2)-1 (methyl-3-piperidino)-1)-1-cyclohexane.

7. Substituted amine according to claim 2, chosen from from among
- (benzo(b)thiophenyl-2)-1 ((ethyleneketal-4 piperidino)-1)-1-cyclohexane,
- (benzo(b)thiophenyl-2)-1((dimethyl-3,5-piperidino)-1)-1-cyclohexane,
- benzo(b)thiophenyl-2)-1(bromomethyl-3-piperidino)-1)-1-cyclohexane,
- benzo(b)thiophenyl-2)-1((iodomethyl-3-piperidino)-1)-1-cyclohexane, and
- (benzo(b)thiophenyl-2)-1 t-methyl-4-r-(piperidino-1)-1-cyclohexane.

8. Process for the preparation of a substituted amine complying with the formula: 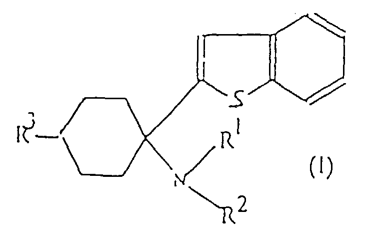 in which R¹ and R², which can be the same or different, represent an alkyl radical or an alkyl radical substituted by at least one substituent chosen from among the halogen atoms, alkoxy radicals and the hydroxy radical, where R¹ and R² form with the nitrogen atom to which they are bonded a piperidine cycle optionally having one or more substituents chosen from among the methyl group, the methyl group substituted by at least one halogen atom and the group 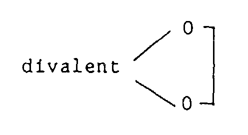 or = 0
or in which R¹ and R² form with the nitrogen atom to which they are bonded a piperazine cycle of formula: 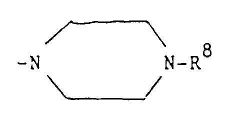 in which R⁸ represents H, an alkyl radical or an alkyl radical substituted by at least one substituent chosen from among halogen atoms and hydroxy, alkoxy, arylalkoxy and oxycarbonylalkyl groups and R³ is a hydrogen atom or the methyl group, provided that R³ does not represent H when R¹ and R² form with the nitrogen atom to which they are bonded an unsubstituted piperidine cycle, characterized in that it consists of:
a) preparing an α-aminonitrile of formula: 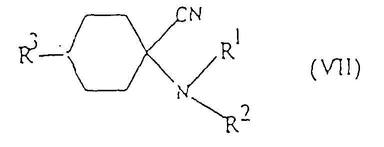 in which R¹, R² and R³ have the meanings given hereinbefore and
b) reacting the thus prepared α-aminonitrile with a halide of formula:
MgXR⁵ in which X represents a halogen atom, except fluorine and R⁵ represents the 2-benzothiophenyl group.

9. Process for the preparation of a substituted amine complying with formula (I): 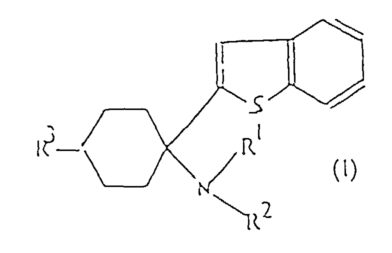 in which R¹ and R² form with the nitrogen atom to which they are bonded a piperidine cycle having one or more substituents constituted by the methyl group substituted by a halogen atom and R³ is a hydrogen atom or the methyl group, characterized in that it consists of:
a) preparing an α-aminonitrile of formula: 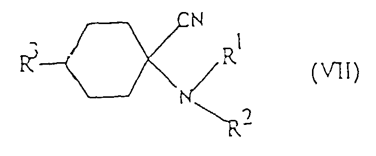 in which R³ has the meaning given hereinbefore and R¹ and R² form with the nitrogen atom to which they are bonded a piperidine cycle substituted by the hydroxymethyl radical,
b) reacting the thus prepared α-aminonitrile with a halide of formula MgXR⁵, in which X represents a halogen atom, except fluorine, and R⁵ represents the 2-benzothiophenyl group and
c) reacting the derivative obtained in stage b) with a halogenating agent or a carboxylic acid.

10. Process according to claim 9, characterized in that the halogenating agent is thionyl bromide, thionyl chloride or trimethyl silane iodide.

11. Process for the preparation of a substituted amine of formula: 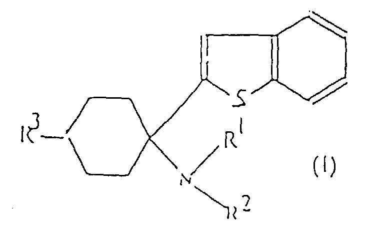 in which R¹ and R² form with the nitrogen atom to which they are bonded a piperidine cycle optionally having one or more substituents chosen from among the methyl group and the methyl group substituted by a halogen atom and R³ is a hydrogen atom or the methyl group, provided that R³ does not represent H when R¹ and R² form with the nitrogen atom to which they are bonded an unsubstituted piperidine cycle, characterized in that it consists of:
a) preparing an alcohol of formula: 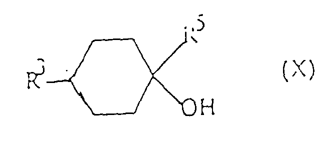 in which R³ has the meaning given hereinbefore and R⁵ represents the 2-benzothiophenyl group,
b) transforming the alcohol prepared in stage a) into a corresponding azide derivative of formula: 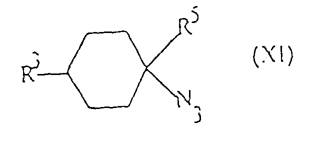
c) reducing the azide derivative into a mixture of primary amines and
d) reacting the mixture of primary amines with a 1,5-dihalogenopentane optionally having one or more substituents chosen from among the methyl group and the methyl group substituted by a halogen atom.

12. Pharmaceutical composition, characterized in that it comprises at least one substituted amine according to any one of the claims 1 to 7, or an addition salt to an acid of said amine.

## Claims (Claims for the following Contracting State(s): GR)

1. Substituted amine complying with the formula: 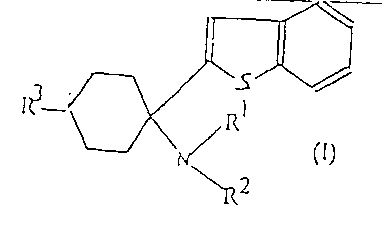 in which R¹ and R², which can be the same or different, represent an alkyl radical or an alkyl radical substituted by at least one substituent chosen from among halogen atoms, alkoxy radicals and the hydroxyl radical, where R¹ and R² form with the nitrogen atom to which they are bonded a piperidine cycle optionally having one or more substituents chosen from among the methyl group, the methyl group substituted by a halogen atom and the group 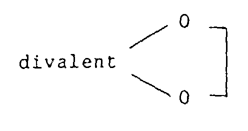 or = 0
in which R¹ and R² form with the nitrogen atom to which they are bonded a piperazine cycle of formula: 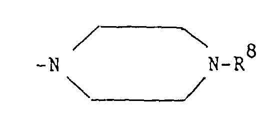 in which R⁸ represents H, an alkyl radical or an alkyl radical substituted by at least one substituent chosen from among halogen atoms and hydroxy, alkoxy, arylalkoxy and oxycarbonylalkyl groups and R³ is a hydrogen atom or the methyl group, provided that R³ does not represent H when R¹ and R² form with the nitrogen atom to which they are bonded an unsubstituted piperidine cycle.

2. Substituted amine according to claim 1, characterized in that R¹ and R² form with the nitrogen atom to which they are bonded an unsubstituted piperidine cycle or a piperidine cycle substituted by at least one methyl group, a methyl group substituted by a halogen atom and/or the group 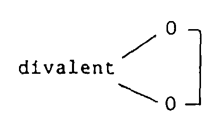 or = 0

3. Substituted amine according to claim 1, characterized in that R¹ and R² are alkyl radicals.

4. Substituted amine according to claim 3, characterized in that it is the (benzo(b)thiophenyl-2)-1 (dipropylamino-1)-1-cyclohexane.

5. Substituted amine according to claim 2, characterized in that it is the (benzo(b)thiophenyl-2)-1 c-methyl-4-r-(piperidino-1)-1-cyclohexane.

6. Substituted amine according to claim 2, characterized in that it is the (benzo(b)thiophenyl-2)-1 (methyl-3-piperidino)-1)-1-cyclohexane.

7. Substituted amine according to claim 2, chosen from from among
- (benzo(b)thiophenyl-2)-1 ((ethyleneketal-4 piperidino)-1)-1-cyclohexane,
- (benzo(b)thiophenyl-2)-1((dimethyl-3,5-piperidino)-1)-1-cyclohexane,
- benzo(b)thiophenyl-2)-1(bromomethyl-3-piperidino)-1)-1-cyclohexane,
- benzo(b)thiophenyl-2)-1((iodomethyl-3-piperidino)-1)-1-cyclohexane, and
- (benzo(b)thiophenyl-2)-1 t-methyl-4-r-(piperidino-1)-1-cyclohexane.

8. Process for the preparation of a substituted amine complying with the formula: 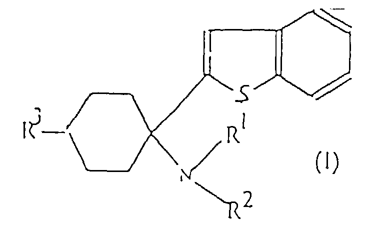 in which R¹ and R², which can be the same or different, represent an alkyl radical or an alkyl radical substituted by at least one substituent chosen from among the halogen atoms, alkoxy radicals and the hydroxy radical, where R¹ and R² form with the nitrogen atom to which they are bonded a piperidine cycle optionally having one or more substituents chosen from among the methyl group, the methyl group substituted by at least one halogen atom and the group 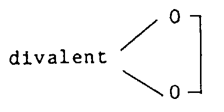 or = 0
or in which R¹ and R² form with the nitrogen atom to which they are bonded a piperazine cycle of formula: 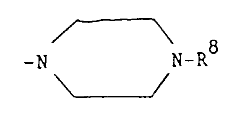 in which R⁸ represents H, an alkyl radical or an alkyl radical substituted by at least one substituent chosen from among halogen atoms and hydroxy, alkoxy, arylalkoxy and oxycarbonylalkyl groups and R³ is a hydrogen atom or the methyl group, provided that R³ does not represent H when R¹ and R² form with the nitrogen atom to which they are bonded an unsubstituted piperidine cycle, characterized in that it consists of:
a) preparing an α-aminonitrile of formula: 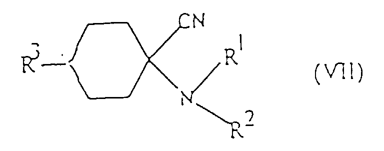 in which R¹, R² and R³ have the meanings given hereinbefore and
b) reacting the thus prepared α-aminonitrile with a halide of formula: MgXR⁵ in which X represents a halogen atom, except fluorine and R⁵ represents the 2-benzothiophenyl group.

9. Process for the preparation of a substituted amine complying with formula (I): 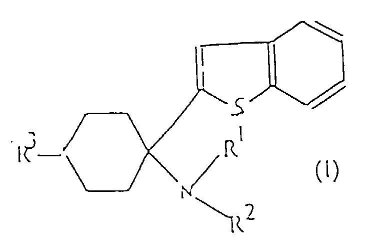 in which R¹ and R² form with the nitrogen atom to which they are bonded a piperidine cycle having one or more substituents constituted by the methyl group substituted by a halogen atom and R³ is a hydrogen atom or the methyl group, characterized in that it consists of:
a) preparing an α-aminonitrile of formula: 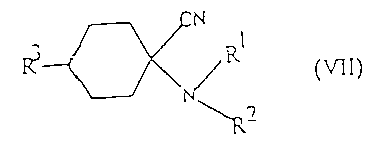 in which R³ has the meaning given hereinbefore and R¹ and R² form with the nitrogen atom to which they are bonded a piperidine cycle substituted by the hydroxymethyl radical,
b) reacting the thus prepared α-aminonitrile with a halide of formula MgXR⁵, in which X represents a halogen atom, except fluorine, and R⁵ represents the 2-benzothiophenyl group and
c) reacting the derivative obtained in stage b) with a halogenating agent or a carboxylic acid.

10. Process according to claim 9, characterized in that the halogenating agent is thionyl bromide, thionyl chloride or trimethyl silane iodide.

11. Process for the preparation of a substituted amine of formula: 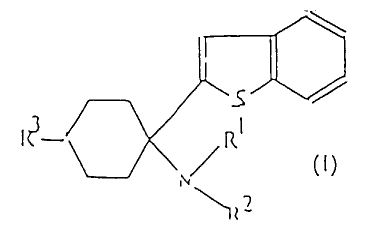 in which R¹ and R² form with the nitrogen atom to which they are bonded a piperidine cycle optionally having one or more substituents chosen from among the methyl group and the methyl group substituted by a halogen atom and R³ is a hydrogen atom or the methyl group, provided that R³ does not represent H when R¹ and R² form with the nitrogen atom to which they are bonded an unsubstituted piperidine cycle, characterized in that it consists of:
a) preparing an alcohol of formula: 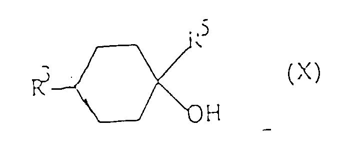 in which R³ has the meaning given hereinbefore and R⁵ represents the 2-benzothiophenyl group,
b) transforming the alcohol prepared in stage a) into a corresponding azide derivative of formula: 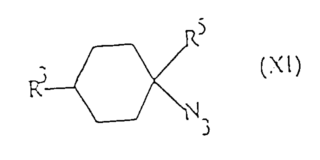
c) reducing the azide derivative into a mixture of primary amines and
d) reacting the mixture of primary amines with a 1,5-dihalogenopentane optionally having one or more substituents chosen from among the methyl group and the methyl group substituted by a halogen atom.

12. Process for the preparation of a pharmaceutical composition, characterized in that it consists of mixing at least one substituted amine according to any one of the claims 1 to 7, or an addition salt to a pharmaceutically acceptable acid of said amine, with a pharmaceutically acceptable diluent or excipient.

## Claims (Claims for the following Contracting State(s): ES)

1. Process for the preparation of a substituted amine complying with the formula: 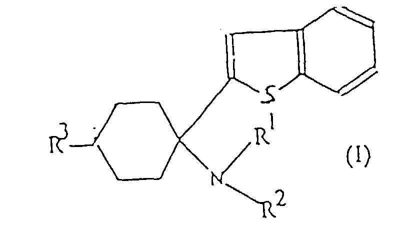 in which R¹ and R², which can be the same or different, represent an alkyl radical or an alkyl radical substituted by at least one substituent chosen from among halogen atoms, alkoxy radicals and the hydroxy radical, where R¹ and R² form with the nitrogen atom to which they are bonded a piperidine cycle optionally having one or more substituents chosen from among the methyl group, the methyl group substituted by at least one halogen atom and the group 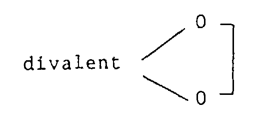 or = 0
in which R¹ and R² form with the nitrogen group to which they are bonded a piperazine cycle of formula: 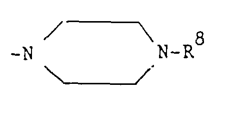 in which R⁸ represents H, an alkyl radical or an alkyl radical substituted by at least one substituent chosen from among the halogen atoms and the hydroxy, alkoxy, arylalkoxy and oxycarbonylalkyl groups and R³ is a hydrogen atom or the methyl group, provided that R³ does not represent H when R¹ and R² form with the nitrogen atom to which they are bonded an unsubstituted piperidine cycle, characterized in that it consists of:
a) preparing an α-aminonitrile of formula: 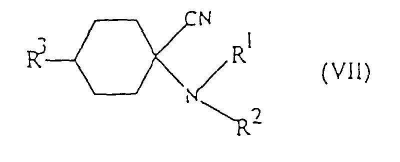 in which R¹, R² and R³ have the meanings given hereinbefore and
b) reacting the thus prepared α-aminonitrile with a halide of formula MgXR⁵, in which X represents a halogen atom, except fluorine, and R⁵ represents the 2-benzothiophenyl group.

2. Process for the preparation of a substituted amine complying with the formula (I): 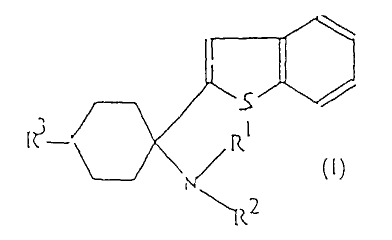 in which R¹ and R² form with the nitrogen atom to which they are bonded a piperidine cycle having one or more substituents constituted by the methyl group substituted by a halogen atom and R³ is a hydrogen atom or the methyl group, characterized in that it consists of:
a) preparing an α-aminonitrile of formula: 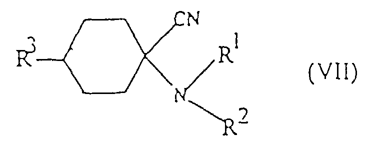 in which R³ has the meanings given hereinbefore and R¹ and R² form with the nitrogen atom to which they are bonded a piperidine cycle substituted by the hydroxy methyl radical,
b) reacting the thus prepared α-aminonitrile with a halide of formula MgXR⁵, in which X represents a halogen atom, except fluorine, and R⁵ represents the 2-benzothiophenyl group,
c) and reacting the derivative obtained in stage b) with a halogenating agent or a carboxylic acid.

3. Process according to claim 2, characterized in that the halogenating agent is thionyl bromide, thionyl chloride or trimethyl silane iodide.

4. Process for the preparation of a substituted amine of formula: 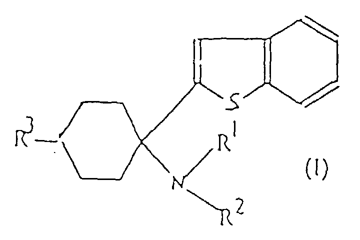 in which R¹ and R² form with the nitrogen atom to which they are bonded a piperidine cycle optionally having one or more substituents chosen from among the methyl group and the methyl group substituted by a halogen atom, R³ is a hydrogen atom or the methyl group, provided that R³ does not represent H when R¹ and R² form with the nitrogen atom to which they are bonded an unsubstituted piperidine cycle, characterized in that it consists of:
a) preparing an alcohol of formula: 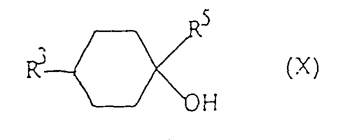 in which R³ has the meaning given hereinbefore and R⁵ represents the 2-benzothiophenyl group,
b) transforming the alcohol prepared in stage a) into a corresponding azide derivative of formula: 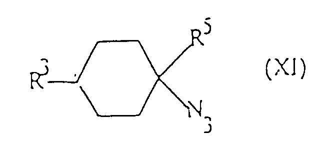
c) reducing the azide derivative into a mixture of primary amines and d) reacting the mixture of primary amines with a 1,5-dihalogenopentane optionally having one or more substituents chosen from among the methyl group and the methyl group substituted by a halogen atom.

5. Process according to claim 1, characterized in that R¹ and R² form with the nitrogen atom to which they are bonded a piperidine cycle either unsubstituted or substituted by at least one methyl group, a methyl group substituted by a halogen atom and/or the group 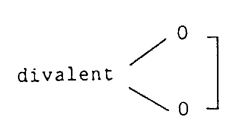 or = 0

6. Process according to claim 1, characterized in that R¹ and R² are alkyl radicals.

7. Process according to claim 6, characterized in that the substituted amine is (benzo(b)thiophenyl-2)-1 (dipropylamino-1)-1-cyclohexane.

8. Process according to claim 2, characterized in that the substituted amine is (benzo(b)thiophenyl-2)-1c-methyl-4-r-(piperidino-1)-1-cyclohexane.

9. Process according to claim 5, characterized in that the substituted amine is (benzo(b)thiophenyl-2)-1 (methyl-3 piperidino)-1)-cyclohexane.

10. Process according to claim 5, characterized in that the substituted amine is chosen from among:
- benzo(b)thiophenyl-2)-1((ethyleneketal-4 piperidino)-1)-1 cyclohexane,
- benzo(b)thiophenyl-2)-1 (dimethyl-3,5 piperidino)-1)-1 cyclohexane,
- benzo(b)thiophenyl-2)-1 ((bromomethyl-3 piperidino)-1)-1 cyclohexane,
- benzo(b)thiophenyl-2-1((iodomethyl-3 piperidino)-1)-1 cyclohexane and
- benzo(b)thiophenyl-2)-1 t-methyl-4-r-(piperidino-1)-1 cyclohexane*.*

11. Process for the preparation of a pharmaceutical composition, characterized in that it consists of mixing at least one substituted amine complying with the formula: 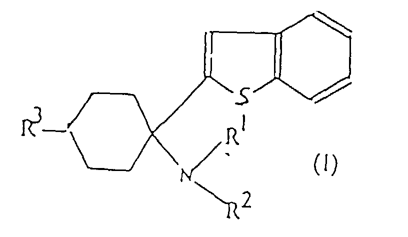 in which R¹ and R², which can be the same or different, represent an alkyl radical or an alkyl radical substituted by at least one substituent chosen from among the halogen atoms, the alkoxy radicals and the hydroxyl radical, where R¹ and R² form with the nitrogen atom to which they are bonded a piperidine cycle optionally having one or more substituents chosen from among the methyl group, the methyl group substituted by a halogen atom and the group 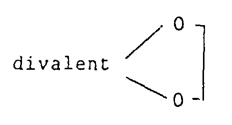 or = 0
or in which R¹ and R² form with the nitrogen atom to which they are bonded a piperazine cycle of formula: 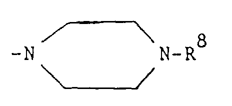 in which R⁸ represents H, an alkyl radical or an alkyl radical substituted by at least one substituent chosen from among the halogen atoms and the hydroxy, alkoxy, arylalkoxy and oxycarbonylalkyl groups and R³ is a hydrogen atom or the methyl group, provided that R³ does not represent H when R¹ and R² form with the nitrogen atom to which they are bonded an unsubstituted piperidine cycle, or an addition salt to a pharmaceutically acceptable acid of said amine, with a pharmaceutically acceptable diluent or excipient.

12. Process according to claim 11, characterized in that the substituted amine is chosen from among:
- (benzo(b)thiophenyl-2)-1 ((ethyleneketal-4-piperidino)-1)-1 cyclohexane,
- (benzo(b)thiophenyl-2)-1((dimethyl-3,5-piperidino)-1)-1 cyclohexane,
- benzo(b)thiophenyl-2)-1((bromomethyl-3-piperidino)-1)-1 cyclohexane,
- benzo(b)thiophenyl-2)-1((iodomethyl-3-piperidino)-1)-1 cyclohexane and
- (benzo(b)thiophenyl-2)-1 t-methyl-4-r-(piperidino-1)-1 cyclohexane.

13. Process according to claim 11, characterized in that the substituted amine is (benzo(b)thiophenyl-2)-1 (dipropylamino-1)-1 cyclohexane.

14. Process according to claim 11, characterized in that the substituted amine is (benzo(b)thiophenyl-2)-1 c-methyl-4-r-(piperidino-1)-1 cyclohexane.

15. Process according to claim 11, characterized in that the substituted amine is (benzo(b)thiophenyl-2)-1 (methyl-3 piperidino)-1)-1 cyclohexane.
